(19) Europäisches Patentamt
European Patent Office
Office européen des brevets

(11) **EP 2 055 715 A1**

## (12) EUROPEAN PATENT APPLICATION

(43) Date of publication:
**06.05.2009  Bulletin 2009/19**

(21) Application number: **07119924.4**

(22) Date of filing: **02.11.2007**

(51) Int Cl.:
*C07K 14/435* (2006.01)    *C12N 15/12* (2006.01)
*C12N 15/63* (2006.01)    *C07K 16/18* (2006.01)
*A61K 38/17* (2006.01)    *A61K 39/395* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **UNIVERSITE LIBRE DE BRUXELLES**
**1050 Brussels (BE)**

(72) Inventors:
• **Beaufays, Jérôme**
 **7090 Braine-le-Comte (BE)**

• **Couvreur, Bernard**
 **1160 Bruxelles (BE)**
• **Godfroid, Edmond**
 **1120 Bruxelles (BE)**
• **Vanhamme, Luc**
 **1490 Court-Saint-Etienne (BE)**

(74) Representative: **pronovem**
**Office Van Malderen**
**Avenue Josse Goffin 158**
**1082 Bruxelles (BE)**

(54) **Anticomplement polypeptides of ixodes ricinus**

(57)    The present invention is related to an isolated and purified polypeptide obtained from tick salivary glands and presenting more than 75% sequence identity with at least a sequence selected from the group consisting of SEQ.ID.NO.1.

FIG. 1

EP 2 055 715 A1

**Description**

**Field of the Invention**

The present invention is related to isolated and purified anticomplement polypeptides of *Ixodes Ricinus* to the nucleotide sequences encoding them and their use for the treatment and/or prevention of cardiovascular disease.

**Background of the invention**

**Tick blood feeding**

Ticks are obligate blood feeding arachnids. They infest many species of mammals, birds, reptiles and amphibians worldwide. They are the vectors of protozoan, bacterial and viral pathogens of prime medical and veterinary importance. Examples of such important pathogens are *Borrelia burgdorferi,* Tick-borne Encephalitis Virus (TBEV), *Babesia bovis* or *Theileria parva,* the respective agents of Lyme disease and viral encephalitis in humans, and babesiosis and theileriosis (East Coast Fever) in cattle. Blood losses due to heavy infestation may weaken the animal, render it more susceptible to other diseases or cause death by exsanguination. In addition, occurrences of tick toxicose including tick paralysis are associated with toxins present in the saliva.

There are two main families of ticks, *Ixodidae* or hard ticks and *Argasidae* or soft ticks. The *Ixodidae* family is further divided into two subdivisions: Prostriata, which contains only the subfamily *Ixodinae,* and Metastriata, which includes the subfamilies *Bothriocrotinae, Amblyomminae, Haemaphysalinae* and *Rhipicephalinae.* Long blood meal by ticks is only possible because these parasites have developed ways to circumvent host defence mechanisms including

 i) haemostasis (coagulation, platelet aggregation and vasoconstriction),
 (ii) inflammatory response, and
 (iii) innate and adaptative immunity.

**Complement Pathway mechanism**

The complement system is a first line of defence against invading pathogens and it links the innate and adaptative responses of the vertebrates' immune system. It consists of a cascade of plasmatic enzymes leading to activation of three effector mechanisms:

 (i) generation of the short potent pro-inflammatory peptides C3a and C5a,
 (ii) deposition of opsonising C3b proteins on cell surfaces,
 (iii) formation of the membrane attack complex (MAC). MACs create pores in the membrane, leading to cell death.

The complement is activated via three main pathways. The classical pathway (CP) is initiated mainly when the C1 complex binds to the Fc region of certain antibody isotypes in immune complexes. The lectin-mediated pathway is activated by the mannose-binding lectin interacting with mannose residues on microbial surfaces. The alternative pathway (AP) is spontaneously activated by hydrolysis of plasmatic C3 in C3 ($H_2O$). C3 ($H_2O$) binds soluble factor B (fB). Bound fB is cleaved by serine protease factor D into the soluble Ba peptide and the larger Bb fragment. The resulting C3 ($H_2O$) Bb complex is the initial C3 convertase. It cleaves fluid-phase C3 into the C3a peptide and the metastable C3b fragment. C3b attaches covalently to a pathogen or cell surface through a short-lived thioester bond. Factor B interacts with C3b, leading to its cleavage by factor D and the formation of the C3 convertase (C3bBb). This complex generates new C3b molecules and amplifies the complement cascade by forming new C3 convertases or C5 convertases (C3b2Bb). The C5 convertase cleaves C5 in C5a and C5b. C5b initiates the formation of the MAC [8].

Host cells are protected from the complement system attacks by plasmatic and membrane-bound regulatory molecules that inactivate complement proteins. The C3 convertases are deactivated by dissociation mediated by surface proteins such as Decay-Accelerating Factor (DAF) and Complement Receptor-1 (CR1), as well as soluble factor H. These proteins bind to C3b and displace Bb [8]. They also act as co-factors for serine protease factor I that cleave C3b [9]. On the other hand, the half-life of the C3 convertase is increased at least 10-fold by properdin [10]. It is present in the plasma as oligomers (dimer, trimer or tetramer) [11,12]. Each monomer is a 53 kDa protein composed of six repetitive thrombospondin domains (TSP), flanked with a N-terminal and C-terminal region [13, 14, 15]. Properdin binds to surface-bound C3b and increases its ability to interact with factor B [16]. It also binds to pre-formed C3 convertases leading to increased stability and preventing inactivation by regulators such as factor H and Factor I [8]. In addition, properdin oligomers attached to C3b on a cell surface interacts with preformed fluid-phase C3b or C3bBb by its others subunits [17]. The essential role of properdin in complement activation has been demonstrated by the capacity of an anti-properdin monoclonal antibody to inhibit activation of the alternative pathway. This monoclonal antibody prevents the interaction between properdin and C3b [18].

AP is the major line of defense against invading pathogens such as bacteria [19]. It is also implicated in guinea pig resistance to the hard tick *Dermacentor andersoni* [20,21]. An inhibitory activity of the alternative pathway of complement has been detected in the saliva or in salivary gland extracts from *Ixodes dammini* [22], *I. hexagonus* and *I. uriae* [23], *I. scapularis* [24] and *I. ricinus* [23, 25]. Valenzuela et al. [24] have purified the active anticomplement component from the saliva of adult *I. scapularis.* N-terminal sequencing combined with the screening of a cDNA library lead to the description of the coding sequence of a tick anticomplement protein named ISAC (*I. scapularis* anti-complement). Recombinant ISAC mimics the anticomplement activity of tick saliva. It interferes with the formation of C3 convertase from C3 and fB, and destabilizes pre-formed C3 convertase. Sequences closely related to ISAC were then cloned by RT/PCR from *I. scapularis* nymphs [26], found by screening a cDNA library with sera from repeatedly infested guinea pigs [27] or by PCR-screening a nymph cDNA library [28]. In *I. pacificus*, sequencing large numbers of cDNA clones from adult salivary glands allowed the discovery of ISAC-I [29]. Finally, using degenerate primers designed from the published Isac sequence, Daix et al. [30] recently cloned the related IRAC I and IRAC II from *I. ricinus.*

In soft ticks too, anticomplement activity is present in the saliva and in salivary gland extracts [31]. In *Ornithodoros moubata,* this activity is due to protein OmCI which inhibits both the alternative and the classical pathways. Its sequence is unrelated to the *Ixodes* anticomplement molecules mentioned above (< 15% amino-acid identity). OmCI binds to component C5 of the complement cascade and belongs to the lipocalin superfamily [32].

The recent characterization of large numbers of cDNA sequences from salivary glands of Ixodid ticks including *I. scapularis* [33,34] and *I. pacificus* [29] indicated that most salivary proteins are expressed as large clusters of related proteins, probably coded by multigenic families. Moreover, genome size and organisation have been examined in *Ixodes scapularis, Boophilus microplus* [35] and *Amblyomma americanum* [36]. The genomes are large: $2.1 \times 10^9$, $7.1 \times 10^9$ bp and $1.04 \times 10^9$ bp respectively. Reassociation rates of genomic DNA indicate that they are composed mainly of moderately repetitive elements, which include transposable elements and members of multigenic families. Therefore organisation in multigenic families is probably a major theme in genome organisation of hard ticks perhaps as an adaptation to bloodfeeding.

## Summary of the invention

The present invention is related to an isolated and purified polypeptide(s) obtained from tick salivary glands and present in more than 40%, 50%, 55%, 60%, 65%, 70%, 75%, 80%, 85%, 86%, 87%, 88%, 89%, 90%, 91%, 92%, 93%, 94%, 95%, 96%, 97%, 98%, 99% sequences identity with SEQ ID.NO.1.

The present invention is also related to a polypeptide sequence comprising or consisting of the sequence SEQ.ID.NO.1, SEQ.ID.NO.2, SEQ.ID.NO.3, SEQ.ID.NO4 or SEQ.ID.NO.5 (IXAC-B1 to IXAC-B5) or a biologically active fragment or portion thereof.

Such biologically active fragment or portion of the sequences SEQ.ID.NO.1 to SEQ.ID.NO.5 means a portion of this sequence which presents the same biological activity as the complete sequence or an antigenic or immunogenic fragment or portion of the sequences SEQ.ID.NO.1 to SEQ.ID.NO.5.

The present invention is also related to a polynucleotide encoding the polypeptide(s) of the present invention or its biologically active (antigenic or immunogenic) fragment(s) or portion(s) thereof.

The polypeptide(s) according to the invention may be modified by or linked to at least one substitution group preferably selected from the group consisting of amide, acetyl, phosphoryl and/or glycosyl groups. Moreover, this polypeptide(s) may take the form of a "mature" protein. They may also be part of larger protein(s) or part of a fusion protein.

Preferably, the polypeptide(s) of the present invention further include(s) at least one additional amino acid sequence which contains secretory or leader sequences, pro-sequences, sequences which help in purification such as multiple histidine residues, or additional sequences for stability during recombination protection.

Another object of the present invention concerns variant(s) of the polynucleotide(s) or the polypeptide(s) of the present invention, a precise definition of this term being given hereafter. Preferably, said variant(s) varie(s) from the referent by conservative amino acid substitutions. Preferably, at least one residue is substituted in said variant with another residue of similar characteristics. Advantageously, the substitutions in said variant are among Ala, Val, Leu and Ile; among Ser and Thr, among the acidic residues Asp and Glu; among Asn and Gln; among the basic residues Lys and Arg; or among aromatic residues Phe and Tyr.

Preferably, in the variant(s) of the present invention, several amino acids are substituted, deleted or added in any combination. Preferably, 5 to 10, more preferably 1 to 5, more preferably 1 to 2 amino acids are substituted, deleted or added in any combination, in this variant (s).

This variant(s) may be a naturally occurring allelic variant(s) of an *Ixodes ricinus* salivary gland polypeptide(s) present in *Ixodes ricinus* salivary glands.

The present invention is also related to a vector comprising at least one element selected from the group consisting of the polynucleotide(s), the polypeptide(s), (the variant(s)), according to the present invention and active fragments thereof.

Another object of the present invention concerns a cell transfected by or comprising the recombinant vector according to the invention.

The present invention further discloses an inhibitor (antisense sequence, ribozyme, antibody, hypervariable portion thereof, nanobody, etc.) raised against the polynucleotide(s), the polypeptide(s), or the portion(s), according to the present invention.

The present invention is also related to an hybridoma cell line expressing said antibody.

Another object of the present invention concerns a pharmaceutical composition comprising adequate pharmaceutical carrier and an element according to the invention and selected from the group consisting of the polynucleotide (s), polypeptide(s), portion thereof variant(s), vector, cell, inhibitor or a mixture thereof. Preferably, said pharmaceutical composition presents immunomodulatory properties.

Another object of the invention is an immunological composition or vaccine for inducing an immunological response in a mammalian host to a tick salivary gland polypeptide which comprises at least one element of the group consisting of

a) a tick salivary gland polynucleotide(s) according to the invention;
b) a tick salivary gland polypeptide(s) according to the invention;
c) possibly the variant(s) according to the invention;
d) epitope-bearing fragments, outer-membrane vesicles or cells (attenuated or otherwise) of components a) or b) or c);
e) and possibly an adequate pharmaceutical carrier or diluent.

The pharmaceutical composition (especially a vaccine) may comprise this various elements of the invention in addition with one or more carrier molecules or one or more adjuvant molecules, anti-oxidants, buffer, bacterio status, solutes thickening agents or ions. Examples of carrier molecules are vectors comprising the polynucleotide sequence according to the invention for a transfection transformation of a cell or a carrier molecule which could be complexed or bounded to one or more of this element. For instance, the isolated protein or polypeptide according to the invention could be bounded to a carrier molecule, such as BSA or hemocyanine for improving its antigenic and immunogenic properties especially for obtaining an efficient vaccinal immune response (humoral and cellular immune response, especially a T-cell immune response).

Furthermore, the isolated nucleotide sequence according to the invention could be bounded to one or more promoter/activator sequence which allows a modulated expression of said nucleotide sequence into specific cells. Vector comprising the isolated nucleotide sequence according to the invention could correspond to plasmids or viral vectors, to cationic vesicles or to other lipid membranes such as liposomes.

This carrier molecules or vectors could be also used as adjuvant for inducing an efficient immune response in a patient especially when the pharmaceutical composition is a vaccine.

The term "adjuvant" has its usual meaning in the art of vaccine technology, i.e. a substance or a composition of matter which is not in itself capable of mounting a specific immune response against the antigen of the vaccine, but which is nevertheless capable of enhancing the immune response against the antigen. In other words, the combination of vaccination with antigen and adjuvant induces an immune response against the antigen which is stronger than that induced by the antigen alone.

Suitable carriers for administration of vaccines are well known in the art and can include buffers, gels, microparticles, implantable solids, solvents, other adjuvants or any other means by which the antigen of the vaccine can be introduced into a subject and be made sufficiently available to produce an immune response to the antigen.

Examples of others adjuvant molecules are saponine or suitable fractions thereof and lipopolysaccharides as described in the document EP 671 948, saponine fractions with one or more sterols present in specific formulation are described in the document WO 2007/068907 in addition.

Other examples of adjuvants are metallic salts, oil in water emulsion, lipid and/or derivative thereof, aminoalkyl glucosaminide phosphate, immunostimulotary oligonucleotides QS21 or combination thereof possibly in association with liposome described in the document WO 2006/123155 or US Patent 6 544 518.

An adjuvant composition may also comprise proteins from the yersinia genus as described in document WO 02/304 58.

An adjuvant could comprise also one or more carrier molecule(s), such as metallic salt particles (aluminium phosphate, aluminium hydroxide, calcium phosphate, magnesium phosphate, iron phosphate, calcium carbonate, magnesium carbonate, calcium sulphate, magnesium hydroxide or double salt like ammonium-iron phosphate, potassium, iron phosphate, calcium iron phosphate, calcium magnesium carbonate or a mixture of these salts or polyporous polymeric particles (such as microbeads or nanoparticles (as described in document WO 02/30458)).

An adjuvant could correspond also to an immuno stimulatory CpG oligo nucleotide, preferably CpG oligo nucleotide having a length between 15 and 45 nucleotides.

The pharmaceutical composition (vaccine) may also comprise other compounds which are used for enhancing the antigenicity or immunogenicity of active compounds by addition of immuno modulators on immuno adjuvants such as a cytokines, interferons, tumor necrosis factors, transforming growth factors, or colony stimulating factors preferably interleukin-2. The immunogenicity of the pharmaceutical composition (vaccine) could be also induced by an adequate immuno adjuvant which is preferably selected from the group consisting of block copolymer, ethylene copolymer, acrylic acid copolymer, an acrylic acid copolymer emulsion, a mineral oil emulsion or a mixture thereof, (such as squalen or squalane).

The pharmaceutical composition (vaccine) of the invention is of any suitable pharmaceutical form. Suitable solid or liquid pharmaceutical forms are, for example, granules, powders, pill, tablets, capsules, suppositories, syrups, emulsions, suspensions, creams, aerosols, drops or injectable solution in ampoule form, in whose preparation excipients and additives such as disintegrants, binders, coating agents, swelling agents, lubricants, flavorings, sweeteners or solubilizers are customarily used. In the particular case of a slow-release composition, the pharmaceutical composition may comprise a biocompatible matrix suitable for slow-release.

Regarding the pharmaceutical carrier, in general, the nature of the carrier will depend on the particular mode of administration being employed. For instance, parenteral formulations usually comprise injectable fluids that include pharmaceutically and physiologically acceptable fluids such as water, physiological saline, balanced salt solutions, or the like as a vehicle. For solid compositions, conventional non-toxic solid carriers can include, for example, pharmaceutical grades of mannitol, lactose, or magnesium stearate. In addition to biologically-neutral carriers, pharmaceutical compositions to be administered can contain minor amounts of non-toxic additives, such as wetting or emulsifying agents, preservatives, and pH buffering agents and the like.

The route of administration of the vaccine or pharmaceutical composition according to the present invention can be any suitable route of administration. It can be topical, intradermal, subcutaneous, oral, intravenous, parenteral, intra-peritoneal.

The medical regime is any suitable regime. Amounts and regimens for the administration of the vaccine or the pharmaceutical composition according to the present invention can be determined by those with ordinary skill in the clinical art of treating the described diseases.

The present invention is also related to the use of the immunological composition, the vaccine, the vector, according to the invention for the manufacture of a medicament to produce antibody and/or T-cell immune response to protect a mammal from bacteria and viruses and related species and in the treatment or the prevention of Lyme diseases or tick encephalitis virus diseases.

The present invention is also related to a method for treating or preventing a disease affecting a mammal, said method comprising the step of administrating to said mammal a sufficient amount of the pharmaceutical composition according to the invention or the immunological composition or vaccine according to the invention, to prevent or cure the transmission of pathogenous agents by tick, especially by *Ixodes ricinus,* or the symptoms of diseases induced by tick or pathogenous agents transmitted by tick.

The present invention is also related to a diagnostic kit for detecting a disease or susceptibility to a disease induced or transmitted by tick, especially *Ixodes ricinus,* which comprises:

a) the tick salivary gland polynucleotide(s) according to the invention or an active fragment thereof;
b) the nucleotide sequence(s) complementary to that a);
c) the tick salivary gland polypeptide(s) according to the invention or an active fragment(s) thereof;
d) possibly the variant(s) according to the invention;
e) the inhibitor (preferably the antibody) according to the invention;
f) a phage displaying the inhibitor (preferably the antibody) according to the invention,

whereby a), b), c), d), e) f) may comprise a substantial component.

The *I. ricinus* salivary gland polypeptides include isolated naturally occurring polypeptides, recombinant polypeptides, synthetic polypeptides, or polypeptides produced by a combination of these methods. Means for preparing such polypeptides are well understood in the art.

When the polynucleotides of the invention are used for the production of an *I. ricinus* salivary gland recombinant polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro-or preprotein sequence, or other fusion peptide portions. For example, a marker sequence, which facilitates purification of the fused polypeptide can be encoded. Preferably, the marker sequence is a hexa-histidine peptide, as provided in the pQE vector (Qiagen, Inc.) and described in Gentz et al.(1989), or is an HA tag, or is glutathione-s-transferase. The polynucleotide may also contain non-coding 5' and 3' sequences, such as transcribed, non-translated sequences, splicing and poly-adenylation signals, ribosome binding sites and sequences that stabilize mRNA.

The present invention further relates to inhibitors being polynucleotides that hybridise preferably stringent conditions to the herein above-described sequences. As herein used, the term "stringent conditions" means hybridisation will occur only if there is at least 80%, and preferably at least 90%, and more preferably at least 95%, yet even more preferably 97-99% identity between the sequences.

The anti-*I. ricinus* salivary gland polypeptide inhibitors, which may be polyclonal antibodies, are prepared by any suitable methods known by the skilled person. Polyclonal antibodies can be raised in a mammal for example, by one or more injections of an immunizing agent and, if desired, an adjuvant. Typically, the immunizing agent and/or adjuvant will be injected in the mammal by multiple subcutaneous or intraperitoneal injections. The immunizing agent may include the *I. ricinus* salivary gland polypeptides portion thereof of the invention or a fusion protein thereof. It may be useful to conjugate the immunizing agent to a protein known to be immunogenic in the mammal being immunized. Examples of such immunogenic proteins or carrier proteins include but are not limited to (keyhole limpet) hemocyanin, serum albumin, (bovine) thyroglobulin, and soybean trypsin inhibitor.

The anti-*I. ricinus* salivary gland polypeptide inhibitors may, alternatively, be monoclonal antibodies. Monoclonal antibodies may be prepared using any suitable methods, for example, hybridoma methods, such as those described by Kohler et al. (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The polynucleotides and polypeptides of the present invention may be employed as research reagents and materials for the development of treatments and diagnostics tools specific to animal and human disease.

Furthermore, the present invention is related to a diagnostic kit for a disease or susceptibility to a disease which comprises:

   a) an *I. ricinus* salivary gland polynucleotide, preferably the nucleotide sequence of one of the gene sequences defined in the sequence listing, or a fragment thereof;
   b) a nucleotide sequence complementary to that of a);
   c) an *I. ricinus* salivary gland polypeptide, preferably the polypeptide encoded by one of the gene sequences defined in the sequence listing, or a fragment thereof;
   d) an inhibitor (antibody) to an *I. ricinus* salivary gland polypeptide or polynucleotide, preferably to the polypeptide encoded by one of the gene sequences defined in the sequence listing; or
   e) a phage displaying an inhibitor (antibody) to an *I. ricinus* salivary gland polypeptide, preferably to the polypeptide encoded by one of the cDNAs sequences defined in the sequence listing; can be prepared by any suitable method. It will be appreciated that in any such kit, a), b), c), d) or e) may comprise a substantial component.

The immunological composition, or vaccine, or the vector, according to the invention, can be used to manufacture a medicament to produce antibody and/or T-cell immune response to protect a mammal from bacteria and viruses and related species and in the treatment or the prevention of Lyme diseases or tick encephalitis virus diseases.

The present invention is also related to the use of the pharmaceutical composition of the invention for the manufacture of a medicament in the treatment or prevention of various diseases in mammals including the human especially in haematology (for the treatment of cardiovascular diseases, especially for improving coagulation clinic in transplantation) (for immunosuppression control in auto-immune diseases, in graft rejection and allergy), in rheumatology (in particular, for the treatment or the prevention of inflammation), in cancer and "in general treatment".

Preferably, an immunological response in a mammal is induced by inoculating the mammal with *I. ricinus* salivary gland polypeptide or epitope-bearing fragments, analogues, recombinant vector, outer-membrane vesicles or cells (attenuated or otherwise), adequate to produce antibody and/or T cell immune response to protect said mammal from bacteria and viruses which could be transmitted during the blood meal of *I. ricinus* and related species. Preferably, the *I. ricinus* salivary gland polypeptides used are those encoded by the cDNAs defined in the sequence listing.

The immunological composition or vaccine have any suitable formulation, and is preferably administered orally or parenterally (including subcutaneous, intramuscular, intravenous, intradermal injection). Suitable parenteral administration formulation include aqueous and non-aqueous sterile injection solutions which may contain anti-oxidants, buffers, bacteriostats and solutes which render the formulation isotonic with the blood of the recipient, and aqueous and non-aqueous sterile suspensions which may include suspending agents or thickening agents.

The formulations may be presented, for example, in unit-dose or multi-dose containers, sealed ampoules and vials and may be stored in a freeze-dried condition requiring only the addition of the sterile liquid carrier immediately prior to use.

The vaccine formulation may also include adjuvant systems for enhancing the immunogenicity to the formulation, such as oil-in water systems and other systems known in the art. The dosage will depend on the specific activity of the vaccine and can be readily determined by routine experimentation.

The present invention will be described hereafter in reference to the enclosed figures presented as non limiting examples of the present invention.

## Detailed description of the invention

Here the inventors completed an inventory of sequences related to *I. scapularis* anticomplement protein ISAC in the salivary glands of *I. ricinus.* The inventors took the opportunity of the discovery of five new sequences to study diversification mechanisms possibly at work on a family of tick salivary proteins (hereby referred to as the IxAC family) and investigated their mechanism of action. The results showed that *I. ricinus* anticomplement proteins specifically bind to properdin, leading to inhibition of the formation of the C3 convertase and arrest of the alternative pathway of complement activation. Sequence diversification is associated to antigenic diversity rather than outstanding divergence of molecular characteristics or activity, host specificity in mammals or stage specificity. This is the first in-depth dedicated analysis of a tick multigenic family. Remarkably, IxACs from *I. ricinus* are also the first inhibitors that specifically target a positive regulator of complement.

While searching for lipocalines homologues in the cDNA from pooled salivary glands of 5 days fed Ixodes ricinus females, the inventors found the fragments of two genes related to the prototypical Ixodes scapularis anticomplement protein ISAC of Valenzuela et al. [24]. The complete coding sequences as well as parts of the 3' and 5' UTR's were then reconstituted by RACE. The new genes (accession numbers: AM407396 and AM407397) coded for two new proteins showing ~ 40 % identity with ISAC and the recently described IRAC I and IRAC II from I. ricinus [30], but over 65 % identity with each other. This led the inventors to suspect the existence of a much larger family of anticomplement proteins in I. ricinus.

Two degenerate primers were designed from the conserved 5' end of the coding sequences of available tick anticomplement proteins. Primer IRI was designed from *I. ricinus* sequences (IRACI, IRACII, AM407396 and AM407397) whereas primer IXO was designed from *Ixodes* spp. sequences (IRACI, IRACII, AM407396 and AM407397, ISAC, Isac-1) (Table 1). They were paired with commercial 3' race primers or with downstream primers designed from the 3' UTR. These primer pairs amplified a single band at ~ 600 pb from two cDNAs prepared from a pool of salivary glands from 70 adult females ticks at day 5 of the bloodmeal.

Two primer pairs were used on cDNA1 and 4 primer pairs were used on cDNA2 making up a total of 6 RT-PCR experiments. The PCR products were inserted into both pCRII and pCDNA3.1V5His, generating a total of 12 independent ligation experiments (supplementary Table S1). 122 clones with inserts of the expected size ($\geq$ 600 bp) were sequenced on both strands. A few of them were disregarded as they coded for proteins unrelated to anticomplement proteins (e.g. ribosomal proteins). A few additional clones with inserts smaller than expected (400 - 500 bp) were sequenced too. They were found to code for homologues of uncharacterized "putative salivary proteins" from *I. scapularis* and *I. pacificus.*

Of the 118 anticomplement-like clones found, most could be assigned to previously described IRAC I, IRAC II, AM407396 and AM407397 on the basis of sequence identity. AM407396 was the most frequent (46.6 %) followed by AM407397 (23.3 %), IRAC I (15.5 %) and IRAC II (0.9%). Three additional new sequences were also identified. They were assigned accession numbers AM407398, AM407399 and AM407400 respectively. They accounted for 3.4 %, 8.6 % and 1.7% of clones, respectively. Sequences found identical in $\geq$ 3 independent clones were considered genuine. AM407400 was only represented by two clones which showed a two nucleotides difference. Therefore, the latter sequence was confirmed independently by amplifying internal fragments with gene-specific primers from salivary gland cDNA. Finally, the same set of sequences was found from the various independent RT-PCR experiments (Table S1). Altogether, the results suggested that a complete or near-complete inventory of IxAC anticomplement messengers from the Ixodes ricinus salivary gland had been achieved.

Nucleotide and peptide sequences of ISAC, IRAC I and AM407396 were used to interrogate databases. A total of 33 entries were recovered (Table S2). They were from *I. scapularis* (30 entries), *I. ricinus* (2 entries) and *I. pacificus* (1 entry). They had been cloned from salivary glands (30) or unspecified tick material (3).

Two sequences containing ambiguous positions and ten sequences with incomplete coding sequences for the mature protein, including Salp9 (AF278574) and Isac-like clone 113 (AY956386), were initially discarded. These 12 entries were from *I. scapularis.* The remaining 21 entries were aligned with our five new sequences from *I. ricinus* (AM407396 to AM407400).

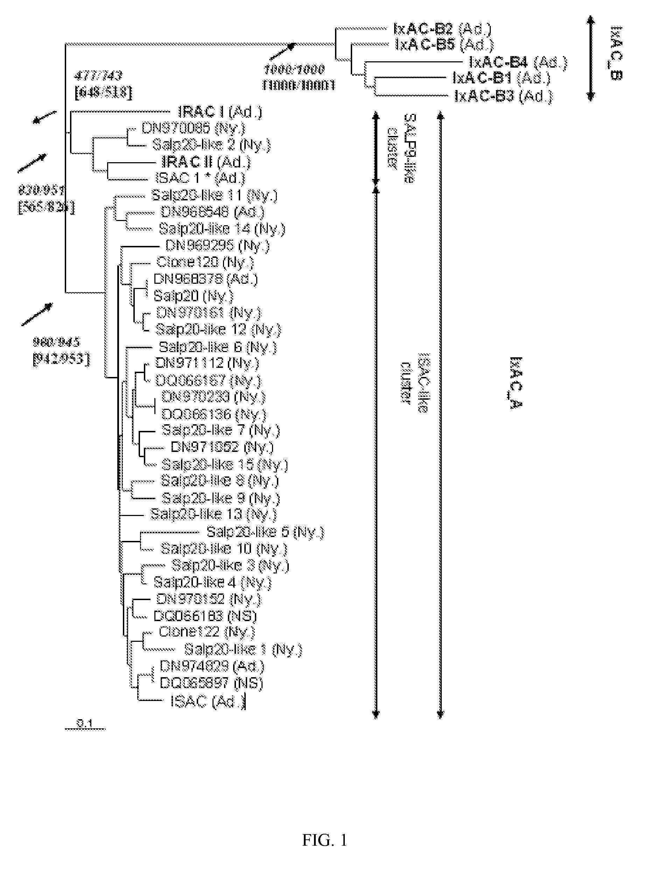

**Figure 1 represents Phylogenetic analysis of *Ixodes* anticomplement proteins.** A distance dendrogramme was constructed from an alignment of 26 tick anticomplement mature proteins using programs in the Phylip 3.65 package (see text). Branch length is proportional to distances between peptide sequences. Indicated near major nodes are bootstrap values, calculated from 1000 replicates of the peptide and nucleotide sequence alignments, respectively. Bold characters: *I. ricinus* entries; *: *I. pacificus* sequence; all others are from *I. scapularis.* Prototypical ISAC is boxed. Sequences are identified by their accession number in databases or by descriptive names when available.

Ad., isolated from adults; Ny., isolated from nymphs; NS, not specified.

All sequences clustered into two main groups or subfamilies, IxAC-A and IxAC-B, which were strongly supported by bootstrap analysis (1000/1000). IxAC-A could be further divided into two clusters: first, a large group containing only *I. scapularis* sequences including prototypical ISAC and secondly, a smaller cluster that contained Isac-1 from *I. pacificus,* IRAC II from *I. ricinus* and EST n° DN970085 from *I. scapularis.* IRAC I could not be joined robustly (bootstrap value <700/1000) to any of the previous two clusters. The IxAC-B subfamily contained our five new sequences AM407396 to AM407400 from *I. ricinus* but none from other tick species. No robust cluster emerged within this subfamily. Clusters were supported by moderate to high bootstrap values (837 to 998/1000).

The maximum-likelihood method was also applied to the initial nucleotide and amino-acid alignment of putative mature proteins. It supported the same topology as with the distance method with slightly different bootstrap values (Fig. 1). The two subfamilies were strongly supported (1000/1000). The two clusters within IxAC-A were also recovered but bootstrap support was moderate ($\sim$ 700/1000 to 998/1000). Again, IRAC I could not be placed in any of the two clusters within IxAC-A.

Only Salp9 grouped with Isac-I, IRAC II and EST n° DN970085 within the second cluster in IxAC-A. Salp9, a 79 residues peptide, aligned to the C-terminal half of DN970085 to which it showed 90% identity. Because ISAC and Salp9 were the earliest tick anticomplement sequences published, the inventors decided to name "ISAC-like" and "Salp9-like", respectively, the large cluster and the small cluster within IxAC-A.

Therefore, the inventors decided to rename the five new sequences (AM407396 to AM407400) IxAC-B1 to IxAC-B5 to indicate the fact that they clustered into the new group or subfamily IxAC-B and away from the previously described IRAC-I and IRAC-II which belong to the IxAC-A subfamily.

Percentages of identity and similarity were calculated for representative IxACs (Table 2). Within a subfamily, amino-acid sequences were over 60 % identical whereas identity dropped to $\sim$ 40 % between the two subfamilies. Within the Isac-like cluster or within the Salp9-like cluster, amino-acid sequences were at least 70% identical.

In summary, phylogenetic analysis of all available tick anticomplement sequences indicated that they robustly segregated into two distinct groups or subfamilies, which the inventors termed IxAC-A and IxAC-B. Intra-group amino-acid identity was > 60% whereas inter-group identity dropped to $\sim$40%. The larger IxAC-A contained sequences from *I. scapularis, I. ricinus* and *I. pacificus.* It could be subdivided into two or possibly three clusters. The smaller IxaC-B contained only these new sequences from *I. ricinus.* At this point of the research no stage-specific group of sequences were identified.

Protein properties.

Properties of the newly discovered proteins were predicted from their amino-acid sequences and compared to prototypical ISAC from *I. scapularis* and related Isac-1 from *I. pacificus.* Calculated PM and pI ranged from 17.46 to 18.03 and 4.01 to 4.29 respectively (Table 3).

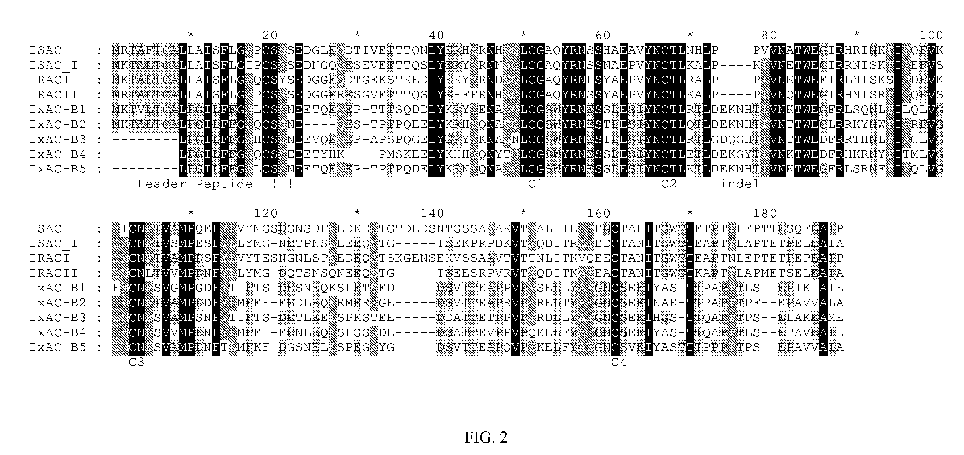

**Figure 2 represents alignment of *Ixodes* anticomplement proteins (SEQ.ID.NO.01 to SEQ.ID.NO.05)**. The 7 anticomplement proteins from *I. ricinus* (IRAC I and II; IxAC-B1 to B5 respectively SEQ.ID.NO.01 to SEQ.ID.NO. 05) were aligned with the prototypical anticomplement protein from *I. scapularis* (ISAC) and the homologue from *I. pacificus* (ISAC_I). Shading indicates the degree of conservation of individual residues. -, gap; ! !, region of predicted signal peptide cleavage; C1 to C4, conserved cysteine residues. Black, dark grey and light grey shadings indicate the degree of conservation of a given residue across the alignement.

All anticomplement proteins presented four conserved cysteine residues predicted to make two disulphide bridges (Figure 2).

Most likely signal peptide cleavage sites for representative IxACs (SEQ.ID.NO.01 to SEQ.ID.NO.05) are indicated in Table 3 and Figure 2. In each individual peptide sequence, SignalP predicted a second or even a third probable, though less likely, cleavage site. They were at position 19 (after C residue), 21 (after SS residues) or 22 (after SSN/E). Altogether, cleavage at any 3 locations within the C↑SS↑(S) ↑E/N motif were theoretically possible.

The presence of a signal peptide and the absence of hydrophobic transmembrane region suggested that they were secreted. This was supported by a TargetP program analysis and confirmed experimentally as recombinant IxACs were recovered in the culture medium after transfection of COS7 or 293T cells.

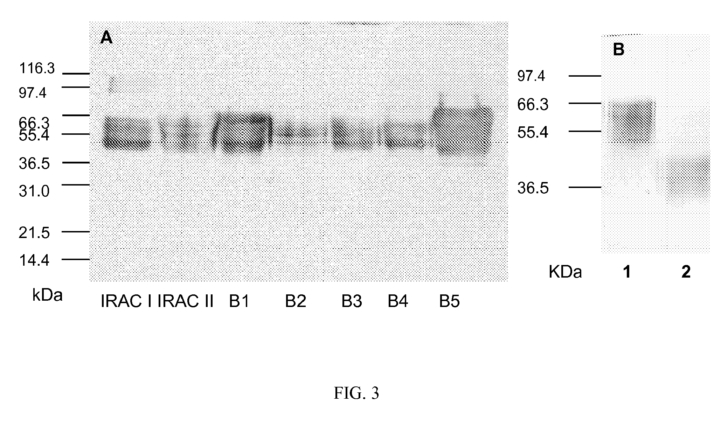

**Figure 3 represents western blot analysis of recombinant IxAC-V5His proteins from *I. ricinus.*** Standardised amounts of recombinant IxAC-V5His proteins from supernatants of transfected 293T cells were analysed by SDS/PAGE and detected by western blotting using an anti-V5 monoclonal antibody. A) parallel analysis of IxACs, B) N-deglycosylation of IxAC-B1-V5His.1, untreated, 2, incubated with PNGase (New England Biolabs).

In a western blot analysis by using an anti-V5 antibody, all recombinant IxACs from *I. ricinus* appear as a series of thin bands at 50-70 kDa (Figure 3a). The apparent molecular weights are consistent with reported values for purified native anticomplement proteins from *I. scapularis* ($\sim$ 65 kDa) [24] and *I. damini* ($\sim$ 49 kDa) [22]. They are in contrast with the predicted MW of $\sim$ 18 kDa. This difference and the aspect of the bands were in agreement with heavy glycosylation.

Indeed, several consensus sites for N- and O- glycosylation were found in the sequences (Table 3). Furthermore, the presence of N-linked glycosylation was experimentally confirmed by treatment with N-glycosidase F that resulted in a drop of the observed MW to 35-45 kDa (Figure 3b). Recombinant Salp20 expressed in insect cells also appear as a smear possibly representing differentially glycosylated forms of the protein [28]. The authors experimentally confirmed the presence of N-linked and O-linked sugars.

Positive (diversifying) selection is operating

Calculation of percentages of identity between the 7 *I. ricinus* anticomplement sequences indicated that they were more closely related at the nucleotide level than at the amino-acid level (Table 2).

A theoretical ancestral sequence was also re-constructed using the Ancescon program. It was aligned to the 7 actual sequences. The numbers of synonymous changes per synonymous sites (dS) and non-synonymous changes per non-synonymous sites (dN) were calculated using the Nei-Gojobori method. Values for dN/dS were consistently > 1 for pairwise comparisons of actual sequences with one another and with the putative ancestral one (Table 4, higher-right triangle). The ratio from overall means of dN and dS values was 2.44. Fisher's exact test for positive selection did not reject the hypothesis of dN > dS except in the case of IRAC I compared to IRAC II (P value < 0.05) (Table 4, lower left triangle).

Therefore all data indicated that diversifying selection was operating within the IxAC family in *I. ricinus.*

Inhibition the alternative pathway (AP), but not the classical pathway (CP) of complement by the IxACs from *I. ricinus.*

The effect of similar amounts of the seven I. ricinus IxACs transiently expressed in 293T cells (Figure 3) were assessed in haemolytic assays of both the classical (CP) and alternative pathways (AP) of complement.

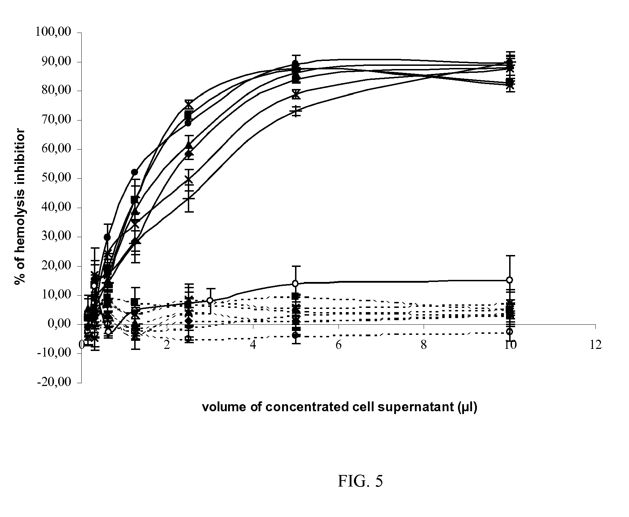

A clear dose-dependent inhibition of the AP was observed for all seven recombinant proteins tested as they inhibited the lysis of rabbit erythrocytes by normal human serum. The shape of the curves and plateau values for inhibition of haemolysis were identical for the seven proteins (Figure 5).

**Figure 5 represents effect of recombinant *I. ricinus* IxAC proteins on the alternative and classical pathways of complement activation.** Assay for the alternative pathway (AP, solid lines) and the classical pathway (CP, dashed lines) of complement activation were conducted in the presence of normalized amounts of recombinant *I. ricinus* IxACs produced in the supernatant of transfected 293T cells. Percent inhibition of rabbit red blood cell lysis in the presence of human serum are indicated. The values are means $\pm$ standard deviation of triplicates. RaHBP2 was used as negative control. ♦:IRAC I; ■:IRAC II; ▲:IxAC-B1; ✕:IxAC-B2; *:IxAC-B3; ●:IxAC-B4; +:IxAC-B5; O:RaHBP2.

The same dilutions of the 7 recombinant proteins were tested again in the CP assay in the presence of normal human serum. As shown in Figure 5, there was no inhibition of lysis of antibody-sensitized sheep erythrocytes. In both assays, no inhibition was observed under addition of recombinant RaHBP2.

Therefore, the inventors concluded that the 7 IxACs from *I. ricinus* similarly inhibited the alternative but not the classical pathway of complement activation.

Inhibition of the cleavage of human C3 and factor B by IxACs

The inventors investigated the effect of *I. ricinus* IxAC proteins on the cleavage of factor B to Bb and on the production of C3a in the AP assay. Supernatants of completed AP assays were analyzed by western blot using antisera to fB or C3a.

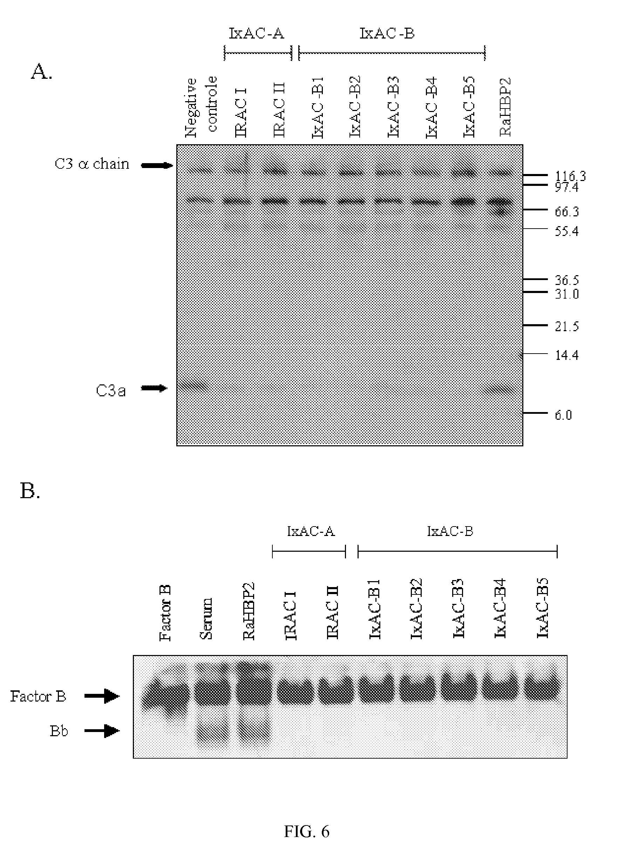

**Figure 6 represents inhibition of C3a formation and factor B cleavage.** Aliquots of supernatant from AP haemolysis assays conducted in the presence of standardized amounts of IxACs from *I. ricinus* and unrelated control RaHBP2 were analyzed by western blotting. Panel A: Blots from gels run under denaturing conditions were probed with monospecific anti-C3a serum. The α-chain of precursor C3 (108 kDa) as well as proteolytic products including the 77 kDa α-chain fragment of iC3(H2O), the 45 kDa autocatalytic α-chain fragment of C3 and the C3a peptide (arrow) are recognized. Panel B: Blots from gels run under non-denaturing conditions were probed with a antiserum to factor B. Purified factor B was used as a positive control.

As shown in Figure 6A, the anti-C3a antibody recognized major bands at 116 kDA, 77kDa and ∼ 10 kDa. Clearly identifiable bands are indicated by arrows, they corresponded to the α-chain of C3 (115 kDa) and the small C3a peptide (9 kDa) [37,38]. The latter is almost completely suppressed in samples from assays run in the presence of recombinant IxACs as compared to sample from assays run in the presence of RaHBP2 or without added protein.

The antiserum to factor B recognized purified factor B as a single band in non-denaturating western blot (Figure 6B). A second band was recognized in samples from control AP assays run in the absence of added protein or in the presence of unrelated RaHBP2. It resolved into two distinct bands presumably corresponding to differently charged forms of Bb [38, 39]. It is absent in sample from AP assays run in the presence of recombinant IxACs.

The inventors concluded that *I. ricinus* IxAC inhibited the formation of C3a and the cleavage of fB. In addition, different members of the two IxAC sub-families showed no detectable differences regarding the extent of inhibition.

*I. ricinus* IxACs specifically interact with properdin.

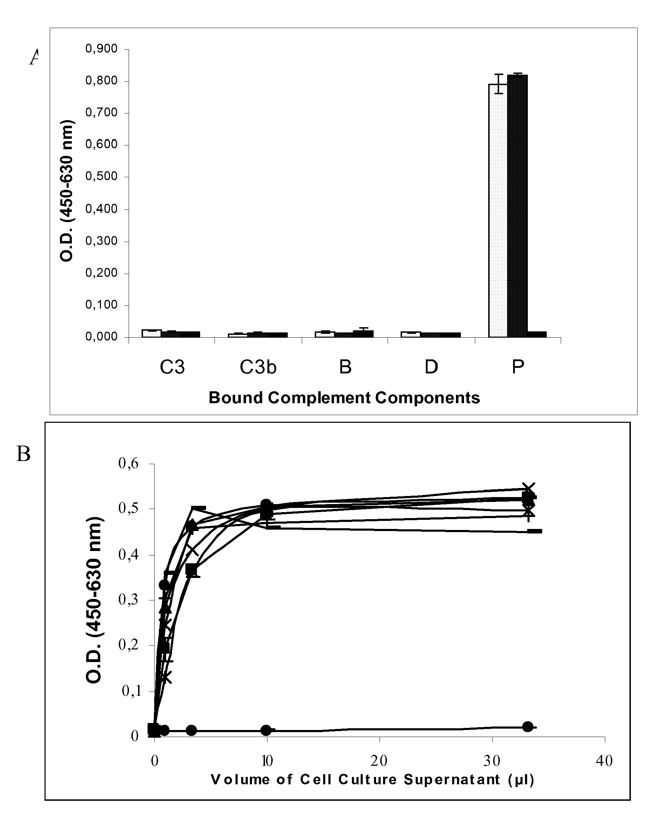

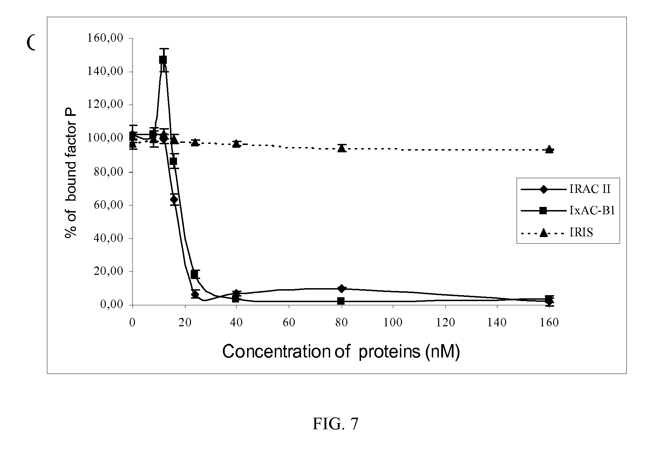

**Figure 7 represents ELISA analysis of the binding of IxAC proteins to immobilized C3 convertase components.** Panel A: Binding of IxACs to components of the AP. Purified recombinant IRAC II, IxAC-B1 or unrelated protein Iris were added to microtiter wells previously coated with purified factors C3, C3b, fB, fD or properdin (P). Bound proteins were detected with an anti-V5 monoclonal antibody using an ELISA format. ⬚: IRAC II; ⬛ : IxAC-B1; ■: Iris. Panel B: Increasing amounts of normalized supernatant from transfected culture 293T cells were added to immobilized properdin. Bound IxACs were detected with an anti-V5 antibody. ♦: Iris; ■: IRAC I; ▲: IRAC II; ×: IxAC-B1; *: IxAC-B2; ●: IxAC-B3; +: IxAC-B4; - : IxAC-B5. Panel C. Competition between properdin and the IxACs for C3b binding. Purified properdin and increasing amounts of IRAC II, IxAC-B1 or unrelated control IRIS were added simultaneously to C3b-precoated microtiter wells. Bound properdin was detected with an anti-properdin monoclonal antibody. ♦: IRAC II; ■: IxAC-B1; ▲: Iris.

The inventors next searched to identify the target or targets of the IxACs using a ELISA methodology. Components of the C3 convertase (i.e. C3, C3b, fB, fD or properdin) were coated to microtitre plates and incubated with recombinant IxAC_V5His. Binding of IxAC was monitored using an anti-V5 antibody. IRAC II and IxAC-B1 purified from the baculovirus/Sf9 expression system, but not unrelated protein Iris, strongly bound to properdin. They did not bind to C3, C3b, fB or fD (Figure 7A). In addition, standardized amounts of the seven *I. ricinus* IxACs but not RaHBP2 bound to properdin in a dose-dependent manner (Figure 7B).

The inventors also tested the binding of properdin to C3b-coated plates in the presence of increasing amounts of IRAC-II, IxAC-B1 and control Iris. Binding was revealed by a monoclonal antibody to properdin (Figure 7C). Increasing amounts of IRAC-II, IxAC-B1 but not Iris lead to a decrease in the amount of bound properdin. The inventors concluded that *I. ricinus* IxACs specifically interacted with properdin and prevented its binding to C3b.

IxAC proteins inhibit the formation of the C3 convertase complex by interacting with properdin.

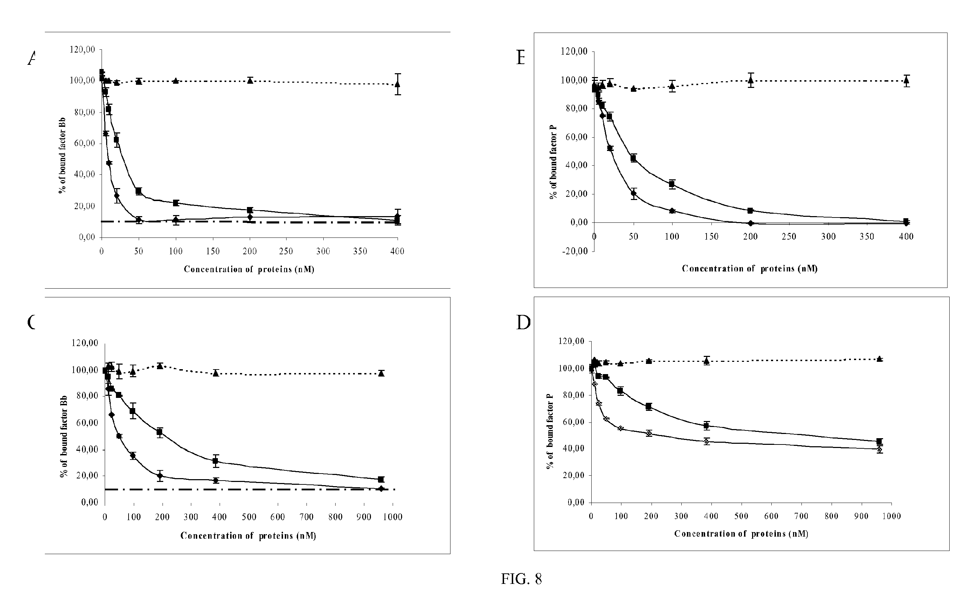

**Figure 8 represents effect of IxAC proteins on the formation and stability of the C3 convertase.** Panel A and B: The effect of IxAC proteins on the formation of C3 convertase was evaluated by incubating simultaneously purified factors B, D and properdin with increasing amounts of IRAC II or IxAC-B1 on C3b-coated wells. Panel C and D: The effect of IxAC proteins on the stability of C3 convertase was assessed by incubating preformed C3 convertase (fB, fD and properdin pre-incubated for 1 hour) on C3b-coated plates with increasing amounts of recombinant IxACs. Bound factor B or properdin were detected with an anti-factor B antibody (A-C) or an anti-properdin antibody (B-D), respectively. Recombinant IRIS was used as negative control. ♦: IRAC II; ■: IxAC-B1; ▲: IRIS.

The inventors also addressed the effect of *I. ricinus* IxACs on the formation and stability of the alternative pathway C3 convertase (C3bBbP). It was reconstituted *in vitro* by adding purified components fB, fD and properdin to C3b-coated plates. Bound Bb and properdin were detected using specific antibodies. The inventors measured ~ 10 times less bound Bb in the absence of properdin than in its presence (Figure 8A).

In a first series of experiments, increasing amounts of purified IRAC II and IxAC-B1 were added together with the individual convertase components to C3b-coated plates. The inventors observed a dose-dependent decrease in the amount of bound Bb (Figure 8A) or properdin (Figure 8B), indicating inhibition of the formation of the complex. At the highest protein concentrations ($\geq$ 200 mM), the amount of bound Bb dropped to values observed when reconstituting the C3 convertase without properdin (Figure 8A). No such effect was observed with protein Iris (Figure 8).

In a second series of experiments, C3bBbP was pre-formed on ELISA plates and then incubated with increasing amounts of IRAC-II and IxAC-B1 proteins and unrelated control Iris (Figure 8C and D). The results indicated that the IxAC proteins induced the displacement of all pre-bound factor Bb (Figure 8C) and about 50 % of pre-bound properdin.

The inventors also performed time-course experiments of C3 convertase formation with properdin (C3bBbP) or without it (C3bBb) in the presence of 200 mM IRAC II, IxAC-B1 or Iris. In the absence of properdin, the amount of bound Bb was much lower than in its presence. In this case, the presence of IxACs or Iris had no effect, indicating that the proteins had no direct effect on the interaction between C3b and Bb. On the contrary, the formation of the C3 convertase in the presence of properdin was strongly affected by IxACs. Values of bound Bb dropped to values observed without properdin (Figure S2).

Taken together, the results indicated that *I. ricinus* IxAC proteins inhibited the formation of the C3 convertase complex by interacting specifically with properdin. They also induced the displacement of pre-bound properdin, and Bb indirectly, in a dose-dependent manner.

Host specificity is observed within Vertebrates.

The inventors next tested the hypothesis that the diversification of anticomplement proteins helps *I. ricinus* to counteract the complement activity from the diverse hosts it may infest. Freshly prepared sera from various vertebrate species were first titrated in the AP assay in order to define the volume causing 50% haemolysis (AH50). A wide range of AH50 values were observed, from the equivalent of 0.25 $\mu$l per micro well test (50 $\mu$l final volume) for *Boa constrictor* to 7.0 $\mu$l per test for Balb/c mice (Table 5). Heat-inactivated samples completely lost the haemolytic activity, confirming that this activity was indeed due to complement.

Identical amounts of normalized IxAC proteins from *I. ricinus* and control RaHBP2 were introduced in the AP haemolysis assay in the presence of AH50 volumes of serum. The 7 anticomplement proteins reproducibly inhibited all mammalian sera in a dose-dependent manner (Table 5 and Figure S3). In some species, such as the human (Figure 3) or B. Taurus and M. musculus (Figure S3) the dose-response curves were similar for the seven IxACs from *I.* ricinus. In other mammals such as, C. familiaris, O. aries, S. domesticus and C. elaphus (Figure S3) lower doses of the proteins had different efficiencies. They did reached similar plateau values at higher doses of protein, though. We also observed that most species were not equally sensitive to IxAC inhibition of the AP. Thus haemolysis of rabbit red blood cells by mouse serum was inhibited at ~ 30% at most whereas the haemolytic activity of human serum was inhibited at ~ 85 %. Intermediate plateau values were observed for the other species tested (Figure S3).

On the contrary, IxACs from I. ricinus did not affect most bird and squamate sera, with the exception of IRAC II and IxAC-B4, which inhibited AP activity of one bird (Phasianus colchicus) and one snake (Elaph guttata), respectively.

In summary, all 7 IxAC from *I. ricinus* inhibited the AP in all mammal species tested. However, some IxACs seems to be more specific of some hosts. Inhibition of the AP in one bird by IRAC-II and one squamate species by IxAC-B4 was also observed.

Antigenic diversification in the IxAC family in *I. ricinus.*

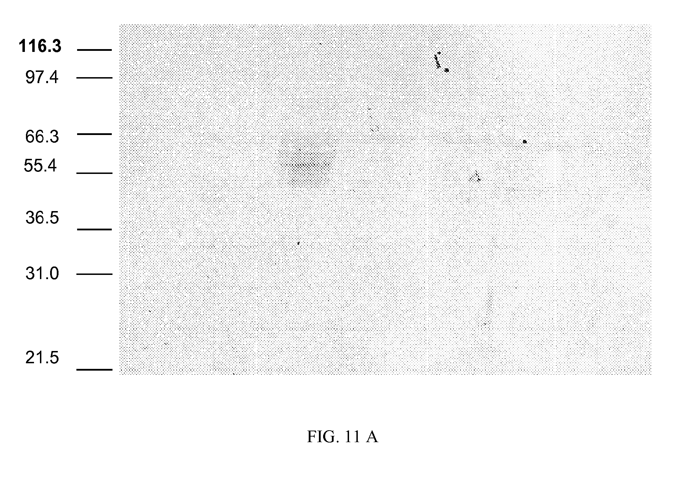

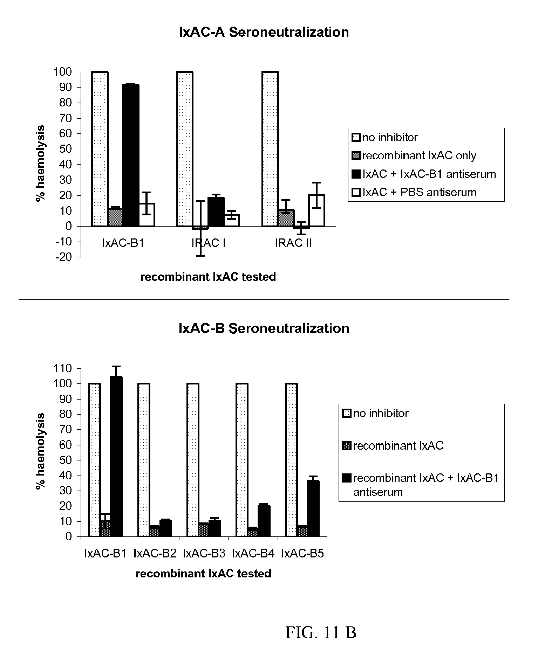

**Figure 11 represents antigenic specificity of recombinant IxACs from *I. ricinus.*** Standardised amounts of the seven *I. ricinus* recombinant IxACs were analysed for antigenic specificity. Panel A. western blot analysis. The serum from a mouse immunised against IxAC-B1 by genetic immunisation followed by a protein boost recognised solely recombinant IxAC B1. M, molecular wesight markers (Mark12, Invitrogen). Panel B. Seroneutralization experiments. AP haemolysis assays were conducted with and without the seven recombinant IxAC. 100% haemolysis was obtained the absence of anticomplement protein ( ). Recombinant IxACs alone ( ) or recombinant IxACs plus heat-inactivated sera from mice immunized against IxAC-B1 (anti IxAC-B1, ■) or mock immunized mice (anti-PBS, ) were added as indicated. Neutralization of activity as indicated by a recovery of haemolysis, was observed only on IxAC-B1. Upper panels: seroneutralization of IxAC-A subfamily. Lower panel: seroneutralization of IxAC-B subfamily. Error bars represent standard deviations.

A monospecific mouse antiserum to IxAC-B1 was produced in mice by DNA immunization followed with a booster with purified recombinant IxAC-B1-V5His. It was used to perform western blot analysis of standardized amounts of IRAC I to IxAC-B5 in parallel with the anti-V5 commercial antibody. As shown in Figure 11A, the anti-IxAC-B1 serum recognized only IxAC-B1 and not any of the other IxACs from *I. ricinus*. This indicated that epitopes recognized on IxAC-B1 in this assay were not present on any other member of the family.

The neutralizing potential of these antibodies was also assessed. Standardized amounts of the 7 recombinant IxACs from *I. ricinus* were pre-incubated with heat-inactivated antiserum to IxAC-B1 before assessing their ability to interfere with complement activity. Neutralization of AP inhibition activity as indicated by a recovery of RBC lysis was observed only against IxAC-B1 (Figure 11B). Seroneutralization of AP inhibition by IxAC-B1 was not observed with pre-immune sera or with antisera directed to the unrelated protein Iris.

Therefore, the inventors concluded that an antiserum raised against one member of the IxAC family is able to recognize and functionally inhibit this member solely and not any other member of the family.

All proteins had very similar biochemical properties. They all inhibited formation of the C3 convertase by specifically binding to properdin. However, diversifying selection was shown to operate. Differences in host specificity or patterns of expression could not account for the sequence diversity. Sequence divergence was associated with antigenic differences. Because individual ticks did not express the same set of anticomplement proteins, that would allow some individuals in a population to escape a host's memory immune reaction.

This is the first time that inhibition of the alternative pathway *via* the specific binding of a positive regulator is described. This is an efficient way to blocking the innate immune system and prevent early rejection of the tick by the host. Recombinant IxACs may thus be useful tools to investigate the role of properdin in physiological and pathophysiological mechanism. Because the alternative pathway of complement activation is implicated in important human pathologies such as ischemia/reperfusion, systemic lupus erythematosus (SLE), asthma and rheumatoid arthritis (RA), inhibitors of the AP may prove to be therapeutically beneficial. To date, only two inhibitors of complement have reached pre-clinical testing: recombinant soluble complement receptor 1 (sCR1) [63] and anti-C5 monoclonal antibodies. However, they do not act specifically on the AP. Selective inhibition of the AP by properdin inhibitors such as IxACs could then be used to treat pathological effects of uncontrolled complement activation due to this pathway, while retaining the classical and lectin ones.

## Experimental procedures.

### Tick material.

Specimens of *Ixodes ricinus* were raised in the tick breeding facility at the *Institut* de *Zoologie, Université* de *Neuchâtel* (Switzerland). Founders of the colony were initially collected in woodlands near Neuchâtel and have been maintained on rabbits (adults and nymphs) and SWISS mice (larvae) for over 20 years. Specimens of *Rhipicephalus appendiculatus* strain Mugaga were a kind gift of Dr Maxime Madder (Animal Health department, Prince Leopold Institute of Tropical Medicine, Antwerp, Belgium). The colony originated from individuals collected in East Africa. It was routinely maintained on rabbits. All specimens were devoid of transmissible pathogens.

Pairs of salivary glands from *I. ricinus* were dissected from

i) 70 adult female specimens at day 5 of the bloodmeal;
ii) 25 females at day 3 of the bloodmeal,
iii) 25 unfed females,
iv) 10 individual females at day 5 of the bloodmeal. Pools of 25 fully gorged larvae and 25 fully gorged nymphs were also prepared. In addition, salivary glands were prepared from 25 adult females and 25 adult males *R. appendiculatus*.

### Nucleic acids extraction and analysis.

PolyA+ RNA was extracted from tick material using the Micro-Fasttrack 2.0 (Invitrogen).

All polyA+ RNA were reverse transcribed with Superscript III (Invitrogen) in the presence of RNaseOUT ribonuclease inhibitor (Invitrogen) using Not1-d(T)18 bifunctional primer (Amersham Biosciences). PolyA+ RNA from pooled day 5 salivary glands was also reverse transcribed with Superscript II (Invitrogen) using the GeneRacer oligodT reverse transcription primer (Invitrogen).

5' and 3' RACE experiments were performed using the Generacer kit (Invitrogen).

For restriction analysis, sequencing and small-scale transfection experiments, recombinant plasmid DNA was extracted using Genelute Plasmid Miniprep Kit (Sigma).

Plasmid constructs were sequenced using universal sequencing primers at Biovallée A.S.B.L. (Gosselies, Belgium) and at Genome Express (Meylan, France).

All commercial reagents and kits were used after the manufacturers' instructions.

PolyA+ RNA extracted from 70 pooled pairs of salivary glands from *I. ricinus* females on day 5 of the bloodmeal was reverse transcribed using two different sets of reverse transcriptase and oligodT primers. cDNA1 was generated with Superscript II using the GeneRacer oligodT reverse transcription primer (Invitrogen). cDNA2 was produced with Superscript III (Invitrogen) in the presence of RNaseOUT ribonuclease inhibitor (Invitrogen) using Not1-d(T)18 bifunctional primer (Amersham Biosciences). Both were used for RT/PCR experiments designed to make an inventory of the family of anticomplement proteins in *I. ricinus.*

Upstream primers were designed manually from the 5' end of coding sequences of anticomplement proteins available early in this project from *I. ricinus* (IRAC I and II, AM407396, AM407397: *IRI* primer) or from all available *Ixodes* spp. sequences (IRAC I and II, AM407396, AM407397, ISAC, Isac-1: *IXO* primer). Commercial downstream primers *Generacer 3'* (Invitrogen) and *Not1* (Amersham Biosciences) are designed to anneal to the 5' end of the modified oligodT primers used for reverse transcription. In addition, downstream primers *UTR1* and *UTR2* were designed from the 3' UTR of anticomplement sequences. All primers are listed in Table 1. They were purchased from Eurogentec (Liège, Belgium) and diluted at 10 μM in MillliQ water.

Six primer pairs were thus constituted:

i) *IRI - generacer3',*
ii) *IXO - generacer3',*
iii) *IRI - Not1,*
iv) *IXO - Not1,*
v) *IXO - UTR1,*
vi) *IXO - UTR2.*

Primer pairs (i) and ii) were used on cDNA1 whereas primer pairs (iii) to (vi) were used on cDNA2 (Table S1). PCR amplification experiments were performed with the Expand High Fidelity Plus PCR System (Roche) in a 50 μl final volume of the commercial buffer containing 1.5 mM MgCl$_2$. PCR cycling parameters were as follows: 2 min denaturation at 94˚C, then 30 cycles of 30 sec at 94˚c, 30 sec annealing at 61 to 65˚C (depending on the Tm of the primer pairs), 1 min at 72˚c, then a final 7 min incubation at 72˚c in a PTC-100 Programmable Thermal Controller (MJ Research). Annealing temperature was set at the lower Tm for a given primer pair minus 5˚c.

PCR products were routinely purified on polyacrylamide gels and inserted into both the pCRII and the pCDNA3.1/V5-His vectors (Invitrogen) by the TA method. Top10F' chemically competent *E. coli* cells (Invitrogen) were then transformed and plated onto LB-Agar-Ampicillin solid medium. Plasmid DNA from recombinant clones was analysed by EcoRI (pCRII) or BamH1 - *Xba*1 (pcDNA3.1/V5-His-TOPO) restriction and inserts ≥600 bp were sequenced on both strands.

Therefore a total of 2 different reverse transcription experiments, 6 different PCR amplifications and 12 ligations were performed during the course of RT/PCR inventories of anticomplement sequences in salivary glands of *I. ricinus* females.

RT-PCR analysis of expression of individual IxACs

Pairs of PCR primers (Table S3) were designed from an alignment of the *I. ricinus* IxACs coding sequences to specifically amplify each of the family members one at a time, each primer pair generating a product of different size. They were synthesized by Sigma-Genosys and purified by HPLC. The specific messengers were searched by RT/PCR in polyA+ prepared from

    i) salivary glands of individual adult females at day 5 of the bloodmeal,

    ii) pooled salivary glands at day 0 of the bloodmeal (unfed females),

    iii) pooled salivary glands of females at day 3 of the bloodmeal,

    iv) pooled nymphs and

    v) pooled larvae.

PCR was performed using Taq polymerase in a 50 μl reaction volume according to the manufacturer's instructions (Roche Biochemicals). Except for IxAC-B4, PCR cycling conditions were as follows: 2 min denaturation at 94˚C then 40 cycles of 30 sec at 94˚C, 30 sec annealing at 57˚C, 30 sec elongation at 72˚C followed by a final 7 min. elongation at 72˚C on PTC-100. This set of PCR conditions had to be modified slightly to amplify the specific IxAC-B4 fragment: 45 cycles and annealing at 60˚C. PolyA+ submitted to reverse transcription without the actual RT enzyme were used as negative control template.

In parallel, family-specific reverse primers were designed from UTR or coding sequences to amplify the full coding sequences of members of IxAC-A (*IXO - CDSREV1*) and IxAC-B (*IXO - CDSREV2*) (Table 1). They were applied to cDNA from pooled nymphs and pooled larvae using the Expand-HF+ system for PCR amplification as described above.

**Expression and quantification of recombinant proteins.**

The coding sequences for I. ricinus IxACS were amplified by PCR and inserted into the vector pCDNA3.1/V5-His-TOPO (Invitrogen). Upstream primers were designed from the first 20 nt of each coding sequences. Nucleotides surrounding the ATG were changed to ACCATGG (IRAC I, IxAC-B1 to B5) or GCCATG (IRAC II) after Kozak's consensus for efficient initiation of translation [60]. Downstream primers were designed from the 3' end of the coding sequences omitting the TGA stop codon so as to create IxAC-V5His chimeras.

The coding sequence for *Rhipicephalus* appendiculatus histamine-binding protein 2 (RaHBP2) was also amplified from salivary gland cDNA of adult R. *appendiculatus* females using PCR primers designed from the original published sequence (U96081) [61] and inserted into vector pCDNA3.1V5His. Throughout this study, recombinant RaHBP2 was used as a negative control.

Subconfluent 293T cells in 35 mm diameter The wells (Orange Scientific) were transfected with 2μg plasmid DNA and 6.0 μl Fugene 6 (Roche Biochemicals) in Dulbecco's modified eagle's medium (DMEM, Invitrogen) without FCS. The medium was harvested after 72h. Pooled supernatants were cleared by centrifugation, concentrated ~50-folds by filtration on 30 kDa cut-off membranes (Millipore), dialyzed against GVB or VB buffers, and finally stored at -80˚c in 40 μl aliquots.

Concentrated culture supernatants were analyzed by western blotting on a Hybond ECL membrane (GE healthcare) using an anti-V5 primary antibody (Invitrogen), an IgHRP conjugate as secondary antibody and the ECL detection reagent (GE healthcare) following the manufacturer's instructions. Autoradiogram signals were quantified with the ImageQuant TL Software (GE Healthcare). After normalization, new western blot analyses indicated protein amount differences of 2.5 fold at most.

Samples were also submitted to N-deglycosylation using N-glycosidase F (New England Biolabs) using conditions recommended by the manufacturer.

The coding region of IRAC II and IxAC-B1 were amplified by PCR (94˚C for 30 s, 56˚C for 30 s, 72˚C for 1 min.; 30 cycles) using the ExTaq DNA Polymerase (Taqara). The PCR product was inserted into the pBlueBac4.5/V5-His Topo vector (Invitrogen) in frame with the coding sequence of the V5 and His epitopes at the C-terminus. Recombinant baculoviruses were generated by recombination between pBlueBac/IxAC and Bac-N-Blue linear DNA virus (Invitrogen). Recombinant viruses were selected and amplified according to the manufacturer's instruction. Sf9 cells were infected with a high-titre stock of recombinant baculovirus and were incubated for 72 hours at 27˚C in Sf900 II Serum-Free Medium (Invitrogen). Recombinant IxACs proteins were purified from the cell culture supernatant by affinity chromatography on a His-Trap column (GE Healthcare). The proteins were recovered in 50 mM NaH2PO4 buffer (pH 7.5) containing 300 mM NaCl and 50 mM of imidazole. In experiments with purified proteins, the inventors used protein Iris, a serpin from the salivary gland of *I. ricinus* [62], as a negative control because it was also expressed in the baculovirus/Sf9 and purified in the same manner.

Computer-assisted analysis of sequences and database interrogations

Probable cellular targeting and eukaryotic leader peptide cleavage site were identified in amino-acid sequences with programs Target P and Signal P respectively [63]. N-linked glycosylation and O-GalNAc (mucin type) glycosylation sites were predicted with programs NetNGlyc 1.0 and NetOGlyc 3.1 [64] respectively. Hydrophobic anchor at the C-terminal end of the peptide sequence was searched with program TMHMM v. 2.0 [65] that predicts transmembrane helices in proteins. All were used online at the Center for Biological Analysis of the Technical University of Denmark (CBS prediction servers: http://www.cbs.dtu.dk/services/). Calculation of molecular and isoelectric point were performed with program Pepstats from EMBOSS online at the European Bioinformatics Institute (http://www.ebi.ac.uk/emboss/pepinfo/). Putative S-S links were searched with program Disulfind [66] at http://cassandra.dsi.unifi.it/disulfind.

Tick anticomplement amino-acid sequences were submitted to Hydrophobic Cluster Analysis (HCA) [67,68]. Briefly, amino-acid sequences in an alignment are compared for the overall distribution of hydrophobic clusters, their sizes, shapes and orientations. They can also be compared for possible particular structures induced by specific residues.

The non-redundant, EST, GSS and PDB databases were interrogated online at the NCBI server (http://www.ncbi.nlm.nih.gov/BLAST/) using programs of the Blast family. Nucleotide and amino acid sequences of ISAC, IRAC I, IxAC-B1 were used as queries. The inventors also interrogated the preliminary releases of the genome projects of the non-*Ixodes* hard ticks *Amblyomma variegatum, Boophilus microplus, Rhipicephalus appendiculatus* online at the Institute for Genomic Research (httpe,//tigrblat.tigr.org/tgi) and A. *americanum* available at the University of Oklahoma cDNA Blast Server (http://www.genome.ou.edu/tick.html).

Phylogenetic analysis

The coding sequences were aligned using ClustalW under the Mega3 package to obtain inframe alignment of codons. Minor manual adjustments were made at the 3' end (C-terminus) of the alignment. The Genedoc package [69] (http://www. psc.edu//biomed/genedoc/) was used to visualise alignments and calculate percent identity of nucleotide and peptide sequences. Percent similarity of peptide sequences was also calculated with Genedoc using a Blosum 62 matrix. For phylogenetic analysis by the distance methods, the inventors used programs in the Phylip 3.65 package (http://evolution.gs.washington.edu/phylip.html). The substitution model was F84 for nucleotide sequences and the JTT matrix for amino-acid sequences distance analysis. The PHYML 2.4.4 package [70] (http://atgc.lirmm.fr/phyml/) was used for maximum likelihood analysis. The inventors used the HKY model for nucleotide sequence analysis and the JTT model for amino-acid sequences analysis. Transition to transversion ratios and proportions of invariable sites were estimated from the dataset. No γ distribution of rates among sites was applied. Bootstrap analysis was performed on 1000 replicates of the initial datasets. Trees were visualized with Treeview 1.6.6 [71].

The inventors used the Ancescon package to reconstruct a probable ancestral coding sequence [72] (http://protevo.eb.tuebingen.mpg.de/toolkit/index.php?view= ancescon) from an alignment of the coding sequences for *I. ricinus* IxAC mature proteins. The dN and dS values were calculated using the Nei-Gojobori method as implemented in the MEGA 3.1 package [73] (http://www.megasoftware.net/).

Serum samples

Complement assays were conducted with freshly prepared sera from mammals, birds and squamates (snakes and lizards) listed in Table 5. All animals were healthy and non-immunised. Except for red deer, they had been maintained in confined environments and there was no evidence or history of tick bites.

Fresh blood samples were left to clot at room temperature for 2 to 6 hours. The sera were separated from the clot by centrifugation. They were then aliquoted and stored at -80˚C. Haemolysed samples were discarded.

Human sera were obtained from four healthy male volunteers, Beagle dog (*Canis familiaris*) sera from three individuals, Sheep (*Ovis aries*) sera from five individuals, Pig (*Sus domesticus*) sera from two individuals, Calf (*Bos taurus*) sera from three individuals, Deer (*Cervus elaphus scoticus*) sera from three individuals. Ten female Balb/c mice (*Mus musculus*) purchased from Harlan Netherlands were bled by retro-orbital puncture. Chicken (*Gallus gallus*) sera were obtained and pooled from four individuals, pheasant (*Phasianus colchicus*) sera from five birds, domestic turkey (*Meleagris gallopavo*) serum from one individual, pigeon (*Columba liva*) sera from five individuals, lizard (*Tropidurus torquatus*) sera from two individuals, snake sera from one individual (*Boa constrictor*) and three individuals (*Elaph guttata*).

Alternative pathway (AP) of complement assay

The recombinant proteins were assessed for inhibition of the alternative pathway of complement (AP) according to [74] on red blood cells (RBC) from naive healthy female New Zealand White rabbits. Briefly, fresh sera were diluted in gelatin-veronal-EGTA buffer (GVB) in micro-wells plates and washed RBCs were added. The surface of rabbit erythrocytes activated the alternative pathway of complement leading to their lysis and release of hemoglobin in the buffer. After 60 min incubation at 37˚c, supernatants were recovered to measure absorbance at 415 nm with a Model 680 microplate reader (Biorad). The volume of serum causing 50% haemolysis (AH50 value) was then determined by serial dilutions and used for further tests.

1 to 10 $\mu$l serum were introduced in the test depending of the host species considered. 100% lysis control consisted in total haemolysis produced by incubating 25 $\mu$l of MilliQ water. Background level (no haemolysis) was determined by incubating the erythrocytes in GVB buffer alone (without added serum). Each experimental point was done in triplicate and experiments were performed at least twice, by different experimentators.

In order to test the inhibitory effect of the new proteins, up to 10 $\mu$l standardized supernatants (see above) were introduced in the AP test. The inhibitor was serially diluted in a final volume of 25$\mu$l GVB in the presence of AH 50 volumes of the host serum under consideration. The assay then proceeded as described above. Percent inhibition of haemolysis was then calculated after:

$$\left[1- \frac{(O.D._{415nm} [\text{serum + inhibitor}] - O.D._{415nm} \text{ GVB control})}{(O.D._{415nm} [\text{serum only}] - O.D._{415nm} \text{ GVB control})}\right] \times 100.$$

Classical pathway (CP) assay

Recombinant proteins were also tested for inhibition of the classical pathway of complement (CP) essentially as described by Colligan [75]. Ready-to-use reagents were purchased from Institut Virion\Serion GmbH (Würtzburg, Germany). They included sheep erythrocytes pre-coated with rabbit anti-sheep RBC antibodies and Veronal Buffer pH 7.3 (VB) containing NaCl, $CaCl_2$ and $MgCl_2$. Briefly, diluted serum was incubated in the presence of antibody-coated sheep RBCs in microplates. Immune complexes on the surface of RBCs activate the classical pathway of complement leading to lysis and release of hemoglobin. As in the AP assay, absorbance at 415 nm in the supernatant is proportional to the amount of RBC that had been lysed. Pooled human serum was first titrated to determine the volume that produces 50 % haemolysis (CH50 value). Starting with 10 $\mu$l, standardized amounts of recombinant proteins were diluted two fold in VB buffer containing the equivalent of 0.8 $\mu$l human serum per test (total volume 25 $\mu$l). Pre-coated sheep erythrocytes were then added and the reaction performed as described above. Results were expressed as percent inhibition of haemolysis as for the AP pathway.

The western blot analysis of fB cleavage and C3a formation in the AP assay.

The generation of peptide C3a from C3 and the cleavage of factor B in human serum during the AP assay in the presence of *I. ricinus* IxACs was assessed by western blot analysis as described by Lawrie et al. [38]. 10μl of supernatant from AP assays conducted in the presence of *I. ricinus* IxAC or control protein RaHBP2 were analyzed by denaturing SDS/PAGE (C3 cleavage) or by non-denaturing PAGE (fB cleavage). Purified factor B was also included as a control in the analysis. Briefly, 10% acrylamide SDS/PAGE in tris -tricine buffer were run according to standard methodology. 10% acrylamide non-denaturing gel electrophoresis was conducted as described except that SDS and β-mercaptoethanol were omitted in all buffers. The material was blotted onto nitrocellulose sheets according to standard methodology. Rabbit antiserum to human C3a (Calbiochem, dilution 1:5000) or goat antiserum to human factor B (Quidel, dilution 1:1000) were used as primary antibodies. Anti-rabbit IgG or Anti-goat IgG horseradish peroxidase conjugates (Promega, dilution 1:5000) were used as secondary antibodies. Blots were developed using the chemiluminescence substrate, ECL+ kit from Amersham (Bucks, UK) in accordance with the manufacturer's instructions. The membranes were then exposed to KODAK X-ray film.

Enzyme-linked Immunosorbent assay for measuring the binding of recombinant IxAC to components of the C3 convertase

The binding of the seven recombinant *I. ricinus* IxAC proteins and control protein RaHBP2 to components of the human C3 convertase was assessed using an enzyme-linked immunosorbent assay (ELISA) format. In some experiments, the inventors also used recombinant IRAC-II, IxAC-B1 and control protein Iris purified from the Baculovirus/Sf9 expression system.

The wells in 96-wells polystyrene microtitre plates (Nunc) were coated with 200 ng purified C3, C3b, factor B, factor D or properdin (Calbiochem) in 100μl pH 7.4 phosphate-buffered saline (PBS, Invitrogen) overnight at 4°C. Wells were washed three times for 5 min. with washing buffer (8.1mM $Na_2HPO_4$; 1.8mM $NAH_2PO_4$; 0.05% Tween 20; 25mM NaCl; 10mM $MgCl_2$) and blocked with PBS-Tween-BSA buffer (1% BSA, 0.1 % tween 20 in PBS pH 7.4) for 1h at 37°C. Increasing amounts of recombinant *I. ricinus* IxACs and control proteins in 50μl sample buffer (8.1mM $Na_2HPO_4$; 1.8mM NAH2PO4; 4% BSA; 0.05% tween 20; 75mM NaCl; 10mM $MgCl_2$) were added to the wells. After incubating for 1h at 37°C, wells were washed 3 times with washing buffer. 100μl antibody buffer (8.1mM $Na_2HPO_4$ ; 1.8mM $NAH_2PO_4$; 4% BSA; 0.05% tween 20; 25mM NaCl; 10mM $MgCl_2$) containing a mouse anti-V5 antibody (Invitrogen, dilution 1:5000) were deposited in wells. The plates were incubated for 1h at 37°C then washed again three times. Horseradish peroxidase-conjugated anti-mouse IgG antibodies (Promega, diluted 1:7500) in 100μl antibody buffer were incubated for 1h to 37°C. After 3 washes, 50μl of 3, 3', 5, 5' -Tetramethylbenzidine (TMB, Sigma) substrate was added. The reaction was stopped by adding 50μl $H_2SO_4$ 0.2 N. Optical density at 450 and 630 nm was measured with a Model 680 microplate reader (Biorad). In a first series of experiments the inventors tested the binding of purified recombinant IRACII and IxAC-B1 and unrelated protein IRIS on components of the C3 convertase. In the second series the inventors tested standardized amounts of the 7 *I. ricinus* IxAC and control protein RaHBP2 as expressed in the supernatant of transfected 293T cells (see above) on purified properdin alone.

The inventors also assessed the influence of recombinant IRACII, IxAC-B1 and unrelated protein IRIS on the binding of Properdin to C3b. wells were coated with 150 ng of C3b. 200 ng factor P and increasing concentrations of IRAC II, IxAC-B1 or unrelated protein IRIS in sample buffer were added simultaneously to the wells. The amount of bound properdin was estimated using a primary mouse monoclonal antibody to factor P (diluted 1:2000; Quidel) and horseradish peroxidase-conjugated anti-mouse IgG antibodies (diluted 1:7500; Promega) using the ELISA methodology described above.

Enzyme -linked immunosorbent assays for assessing the Effect of IxACs on the formation and stability of the C3 convertase.

The inventors assessed the effect of recombinant IxAC proteins on the formation and on the stability of the C3 convertase *in vitro* using the ELISA format described above. Polystyrene microtiter 96-wells plates were first coated with 150 ng of C3b per wells. The effect of IxAC proteins on the formation of the C3 convertase was assessed by adding 200 ng of factor B, 20 ng of factor D, 200 ng of factor P in sample buffer to the The inventorslls together with increasing concentrations of IRAC II, IxAC-B1 or IRIS. The plates were incubated for one hour at 37°c before antibody detection of fB or P. To assess the effect of IxACs on the stability of the C3 convertase, C3b-coated wells were incubated for 1 h with 200 ng of factor B, 20 ng of factor D, 200 ng of factor P in sample buffer. The wells were washed three times before deposition of IRAC II, IxAC-B1 or IRIS proteins. The plates were further incubated for one hour at 37°C. Anti-factor P (dilution 1:2000) and anti-factor B (dilution 1:1000) were used as primary antibody to detect bound proteins as described above. Results are expressed as % bound fB or properdin after the following formula:

$$[1 - \frac{(OD\ max - OD\ b) - (OD\ s - OD\ b]}{(OD\ max - OD\ b)}] \times 100.$$

OD b = OD background

OD s = OD sample

Background values are obtained when measuring OD values of C3-coated plates revealed with anti-fB or anti-properdin antibodies. OD max is measured when performing the test without added IxAC.

In a complementary experiment, The inventors compared the effect of IRAC-I, IxAC-B1 and control protein Iris on the formation of the C3 convertase in the presence (C3bBbP) or in the absence of properdin (C3Bb). 200ng of factor B, 20ng of D, with or without 20ng of properdin, and 200 ng of IRAC-I, IxAC-B1 or control protein Iris were added to microtitre wells that had been pre-coated with 150ng purified C3b. The reaction was stopped at 0, 20, 40 and 60 min. and bound factor B or properdin were detected with the respective specific antibodies in the ELISA format described above.

Immunization and antigenic assay.

Antibodies specific to IxAC-B1 were produced in balb/c mice by DNA immunization followed by a booster protein injection. Briefly, 100 $\mu$g plasmid pCDNA3.1/IxAC-B1_V5-His DNA in saline was injected four times in the anterior tibialis at three week intervals. The animals were then boosted once with 1 $\mu$g purified IxAC-B1 in Alum (Brenntag Biosector, DK). Sera were taken by retro-orbital puncture two to 5 weeks after the boost. Recombinant IxAC-B1_V5His protein had been produced by transfecting subconfluent 293T cells with plasmid pCDNA3.1/IxAC-B1V5His in serum-free culture medium. The recombinant protein was purified from cell culture supernatants by chromatography on NiNTa columns (Invitrogen). Pre-immune and immune antisera were tested by western blot on the 7 *I. ricinus* IxACs and assessed *in vitro* for neutralization of anticomplement activity in the AP test. The inventors reasoned that if seroneutralisation occurred, antibodies would block inhibition of AP by IxACS and haemolysis would appear. 3.0 $\mu$l heat-inactivated mouse antisera were preincubated 30 min at room temperature with 2.0 $\mu$l samples of standardized IxACS. The mixture was then added to 2.0 $\mu$l human serum in GVB buffer in a total volume 25 $\mu$l. 25 $\mu$l RBC were then added and the AP assay was conducted as described above. Controls included

i) sera from mouse previously mock-immunised 3 times with PBS in Freund's adjuvant,

ii) serum from mice immunized with the unrelated protein Iris [62],

iii) GVB buffer. Results are expressed as % haemolysis as:

$$\frac{(DO_{415}\ sample - DO_{415}\ GVB)}{(DO_{415}\ serum - DO_{415}\ GVB)} \times 100$$

**References**

8. Janeway CA Jr, et al. MJ Immunobiology 5th ed., Garland Publishing (2001).

9. Hourcade D, et al., Adv Immunol 45:381-416 (1989).

10. Fearon DT, Austen KF J Exp Med. 142:856-63 (1975).

11. Pangburn MK J immunol 142:202-207 (1989).

12. Smith CA, et al. J Biol Chem 259:4582-4588 (1984).

13. Goundis D, Reid KBM. Nature 335:82-85 (1988).

14. Nolan KF, et al. Eur J Immunol 21:771-776 (1991).

15. Smith KF, et al. Biochemistry 30:8000-8008 (1991).

16. DiScipio RG. Biochem J. 199:485-496 (1981).

17. Hourcade DE. J Biol Chem 281:2128-2132 (2006).

18. Gupta-Bansal R, et al. Mol Immunol. 37:191-201 (2000).

19. Joiner KA. Ann Rev Microbiol 42:201-230 (1988).

20. Wikel SK, Allen JR. Immunology 32:457-465 (1977).

21. Wikel SK. Am J Trop Med Hyg. 28:586-590 (1979).

22. Ribeiro JM. Exp Parasitol. 64:347-353 (1987b).

23. Lawrie CH, et al. Exp Parasitol 93:207-214 (1999).

24. Valenzuela JG, et al. J Biol Chem 275:18717-18723 (2000).

25. Mejri N, et al. Parasitol Res 88:192-197 (2002).

26. Soares CA, et al. Insect Mol Biol 14:443-452 (2005).

27. Das S, FS, et al. J Infect Dis 184:1056-1064 (2001).

28. Tyson K, et al. Insect Molecular Biology 16: 469-479 (2007).

29. Francischetti IM, JF, et al. Insect Biochem Mol Biol. 35:1142-1161 (2005).

30. Daix V, et al. Insect Mol Biol. 16:155-166 (2007).

31. Astigarraga A, et al. Parasite Immunol. 19:401-410 (1997).

32. Nunn MA, et al. J Immunol 174:2084-2091 (2005).

33. Valenzuela JG, et al. J Exp Biol 205(Pt 18):2843-2864 (2002).

34. Ribeiro JM, et al. Insect Biochem Mol Biol 36:111-129 (2006).

35. Ullmann AJ, et al. Insect Mol Biol 4:217-222 (2005).

36. Palmer MJ, et al. Insect Mol Biol 3:57-62 (1994).

37. Tack BF, et al. Academic Press, New York 1981. p.64 (1981).

38. Lawrie CH, et al. Mol Immunol. 42:31-38 (2005).

39. Curman B, et al. Biochemistry. 16:5368-5375 (1977).

40. Kordis D, et al. Gene. 261:43-52 (2000).

41. Zupunski V, et al.FEBS Lett 547:131-136 (2003).

42. Mans BJ, et al. Mol Biol Evol 19:1695-1705 (2002).

43. Lanzaro GC, et al. Insect Mol Biol. 8:267-275 (1999).

44. Milleron RS, et al. Am J Trop Med Hyg 70:286-293 (2004a).

45. Milleron RS, et al. Am J Trop Med Hyg 70:278-285 (2004b).

46. Schreiber RD, et al. J Exp Med 148:1722-1727 (1978).

47. Pangburn MK, et al. Biochem J 235:723-730 (1986).

48. Finlay BB, et al. Cell. 124:767-782 (2006).

49. Kostavasili I, et al. J Immunol. 158:1763-1771 (1997).

50. Chung KM, et al. Proc Natl Acad Sci U S A. 103:19111-191116 (2006).

51. Reid KB & Day AJ. Immunol. Today 10:177-180 (1989).

52. Anderson JF, Scand J Infect Dis - Suppl. 77: 3-34 (1991).

53. Milne A, Parasitology 39:173-197 (1949).

54. Hoogstraal H, Aeschlimann A. Bulletin de la société entomologique suisse 55:5-32 (1982).

55. Schroeder H, et al. Microbes Infect 9:247-250 (2007).

56. Nonaka M, Kimura A, Immunogenetics 58:701-713 (2006).

57. Holers V.M., Thurman JM, Mol Immunol 41(2-3):147-52 (2004).

58. The inventorsisman HF, et al. Trans Assoc Am Physicians 103:64-72 (1990).

59. Thomas TC, et al. Mol Immunol 33:1389-401 (1996).

60. Kozak M. J Cell Biol 108:229-241 (1989).

61. Paesen GC, et al. Mol Cell 3:661-671 (1999).

62. Prevot PP, et al. J Biol Chem. 281:26361-26369 2006).

63. Emanuelsson O, et al. Protocols 2:953-971 (2007).

64. Julenius K, et al. Glycobiology 15:153-164 (2005).

65. Krogh A, et al. J Mol Biol 305:567-580 (2001).

66. Vullo A, et al. Bioinformatics 20:653-659 (2004).

67. Gaboriaud C, et al. FEBS Lett 224:149-155 1987).

68. Callebaut I, et al. Cell Mol Life Sci. 53:621-645 (1997).

69. Nicholas KB, et al.EMBNEW NEWS 4:14 (1997).

70. Guindon S, et al. Systematic Biology 52:696-704 (2003).

71. Page RD. Comput Appl Biosci. 12:357-358 (1996).

72. Cai W, Pei J, Grishin NV, BMC Evol Biol. 4:33 (2004).

73. Kumar S, et al. Brief Bioinform. 5:150-163 (2004).

74. Giclas PC, et al. Manual of clinical laboratory immunology, 5th edition, ASM Press, Washinton DC. pp. 181-186 (1997).

75. Colligan JE. Wiley/Interscience, New-York. pp. 13.1.1 - 13.2.7 (1994).

**Claims**

1. An isolated and purified polypeptide obtained from tick salivary glands and presenting more than 60% sequence identity with at least a sequence selected from the group consisting of SEQ.ID.NO.1.

2. The polypeptide of claim 1, which presents at least 70% sequence identity with SEQ.ID.NO.1.

3. The polypeptide according to claim 1 or 2, which presents at least 80%, sequence identity with SEQ.ID.NO.1.

4. The polypeptide according to any of the preceding claims 1 to 3, which presents at least 90% sequence identity with SEQ.ID.NO.1.

5. The polypeptide according to any of the preceding claims 1 to 4, which presents at least 95% sequence identity with SEQ.ID.NO.1.

6. The polypeptide according to any of the preceding claims 1 to 5, which presents at least 99% identity with SEQ.ID.NO. 1.

7. A polypeptide comprising or consisting of the sequence SEQ.ID.NO.1 to SEQ.ID.NO.5 active fragment thereof.

8. The polypeptide according to claims 1 to 7 modified by or linked to at least one substitution group, preferably selected from the group consisting of amide, acetyl, phosphoryl, and/or glycosyl groups.

9. The polypeptide according to any of the preceding claims 1 to 8 further including at least one additional amino acid sequence which contains secretory or leader sequences, pro-sequences, sequences which help in purification such as multiple histidine residues, or additional sequences for stability during recombination protection.

10. A polynucleotide sequence encoding the polypeptide according to any of the preceding claims 1 to 9.

11. A vector comprising at least one element selected from the group consisting of the polypeptide according to any of the claims 1 to 9 or the polynucleotide of claim 10.

12. A cell transfected or comprising the vector according to claim 11.

13. An antibody or an hypervariable fragment thereof raised against the polypeptide according to any of the claims 1 to 9 or the polynucleotide according to the claim 10.

14. A hybridoma cell line expressing the antibody according to claim 13.

15. A pharmaceutical composition comprising an adequate pharmaceutical carrier and an element selected from the group consisting of the polypeptide according to any of the claims 1 to 9, the polynucleotide according to the claim 10, the vector according to claim 11, the cell according to claim 12, the antibody according to claim 13, or a mixture thereof.

16. An immunological composition or vaccine for inducing an immunological response in a mammalian host to a tick salivary gland polypeptide which comprises at least one element of the group consisting of:

    a) the tick salivary gland polypeptide according to any of the claims 1 to 9;

b) the tick salivary gland polynucleotide according to any of the claim 10;
c) epitope-bearing fragments, analogs, outer-membrane vesicles or cells (attenuated or otherwise) of components a) or b);
d) possibly an adequate pharmaceutical carrier.

17. Use of the immunological composition or vaccine according to claim 16 or the vector according to claim 11 for the manufacture of a medicament to produce antibody and/or T-cell immune response to protect a mammal from bacteria and viruses and related species and in the treatment or the prevention of Lyme diseases or tick encephalitis virus diseases.

18. Use of the pharmaceutical composition according to claim 15 for the manufacture of a medicament in the treatment or the prevention of a disease selected from the group consisting of cardiovascular disease, cancer, inflammation, graft rejection, auto immune diseases or allergy.

19. A diagnostic kit for detecting a disease or susceptibility to a disease induced or transmitted by tick, especially *Ixodes ricinus,* which comprises:

a) the tick salivary gland polypeptide according to the claims 1 to 9;
b) the tick salivary gland polynucleotide according to the claim 10;
c) the antibody according to claim 13;
d) a phage displaying the antibody according to claim 13.

FIG. 1

```
               *         20         *         40         *         60         *         80         *        100
ISAC     : MRTAFTCALLAISFLG.PCS.SEDGLE.DTIVE.TTQNLYERH.RNH...LCGAQYRNSSHAEAVYNCTLNHLP----PVVNATWEGIRHRINK.I.QFVK
ISAC_I   : MKTALTCALLAISFLGIPCS.SEDNGQ.ESEVE.TTQSLYERY.RNN...LCGAQYRNSSNAEPVYNCTLKALP----K.VNETWEGIRRNISK.I.EFVS
IRACI    : MKTALTCALLAISFLG.QCSYSEDGGE.DTGEKSTKEDLYEKY.RND...LCGAQYRNLSYAEPVYNCTLRALP----P.VNKTWEEIRLNISKSI.DFVK
IRACII   : MRTALTCALLAISFLG.PCS.SEDGGERESGVE.TTQSLYEHFFRNH...LCGAQYRNSSYAEPVYNCTLKALP----P.VNQTWEGIRHNISR.I.QFVS
IxAC-B1  : MKTVLTCALFGILFFG.LCS.NEETQE.EP-TTTSQDDLYKRY.ENA...LCGSWYRNESSLESIYNCTLRTLDEKNHT.VNKTWEGFRLSQFL.ILQLVG
IxAC-B2  : MKTALTCALFGILFFG.QCS.NE----ES-TPTPQEELYKRH.QNA...LCGSWYRNESTLESIYNCTLQTLDEKNHT.VNTTWEGLRRKYIW.I.RFVG
IxAC-B3  : --------LFGILFFG.HCS.NEEVQE.EP-APSPQGELYKRY.KNA.NLCGSWYRNESILESIYNCTLRILGDQGHT.VNTTWEDFRRTHNL.I.GLVG
IxAC-B4  : --------LFGILFFG.QCS.EEETYHK----PMSKEELYKHH.QNYT.LCGSWYRNESSLESIYNCTLETLDEKGYT.VNKTWEDFRHKRKY.ITMLVG
IxAC-B5  : --------LFGILFFG.LCS.NEETQE.EP-TPTPQDELYKRN.QNA...LCGSWYRNESSLESIYNCTLKTLDEKNHT.VNKTWEGFRLSRNF.I.QLVG
              Leader Peptide  ! !                            C1              C2        indel

               *        120         *        140         *        160         *        180
ISAC     : ICN.TVAMPQEF.VYMGSDGNSDF.EDKE.TGTDEDSNTGSSAAAKVT.ALIIE.E.CTAHITGWTTETPT.LEPTTESQFFAIP
ISAC_I   : .CN.TVSMPESF.LYMG-NETPNS.EEEQ.TG-----TSEKPRPDKVT.QDITR.EDCTANITGW.TEAPT.LAPTETPELEATA
IRACI    : .CN.TVAMPDSF.VYTESNGNLSP.EDEQ.TSKGENSEKVSSAAVTVTTNLITKVQEECTANITGWTTEAPTNLEPTETPEPEAIP
IRACII   : .CNLTVVMPDNF.LYMG-DQTSNSQNEEQ.TG-----TSEESRPVRVT.QDITK.EACTANITGWSTKAPT.LAPMETSELEAIA
IxAC-B1  : F.CN.SVGMPGDF.TIFTS-DESNEQKSLET.ED-----DSVTTKAPPVP.SELLY.GNCSEKIYAS-TTPAP.TLS--EPIK-ATE
IxAC-B2  : .CN.TVAMPDDF.MFEF-EEDLEQ.RMER.GE-----DSVTTKAPRVP.RELTY.GNCSEKINAK-TTPAP.TPF--KPAVVALA
IxAC-B3  : .CN.SVAMPSNF.TIFTS-DETLEE.SPKSTEE-----DDATTETPPVP.RDLLY.GNCSEKIHGS-TTQAP.TPS--ELAKEAME
IxAC-B4  : .CN.SVVMPDNF.MFEF-EENLEQ.SLGS.DE-----DSATTEVPPVPQKELFY.GNCGEKIYAS-TTQAP.TLS--ETAVEAIE
IxAC-B5  : .CN.SVAMPDNFT.MFKF-DGSNEL.SPEG.YG-----DSVTTEAPQVP.KELFY.GNCSVKIYASTTTPPP.TPS--FPAVVAIA
              C3                                        C4
```

FIG. 2

FIG. 3

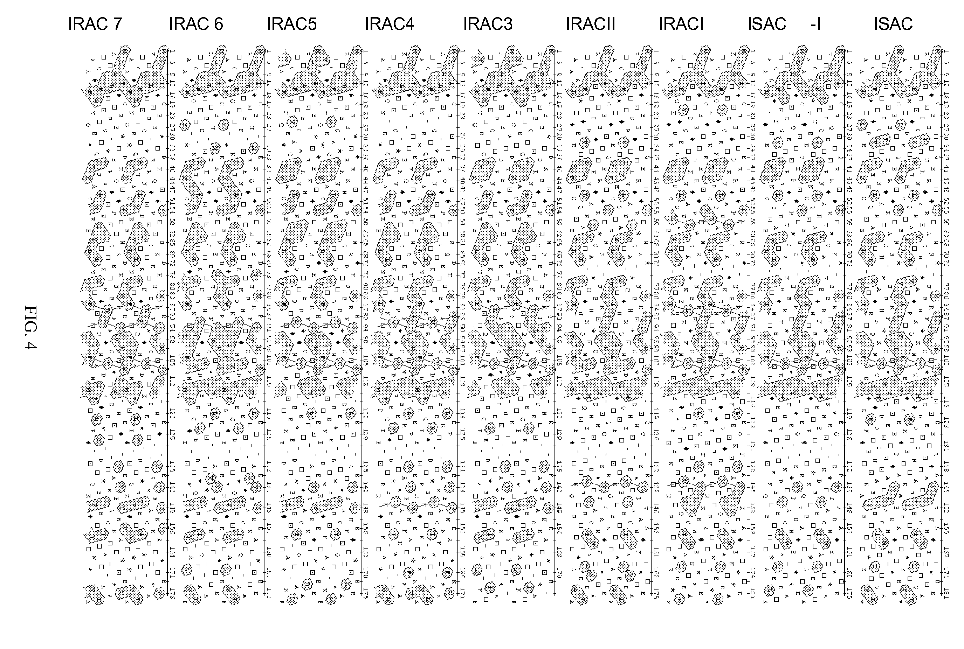

FIG. 4

FIG. 5

A.

B.

FIG. 6

FIG. 7

FIG. 8

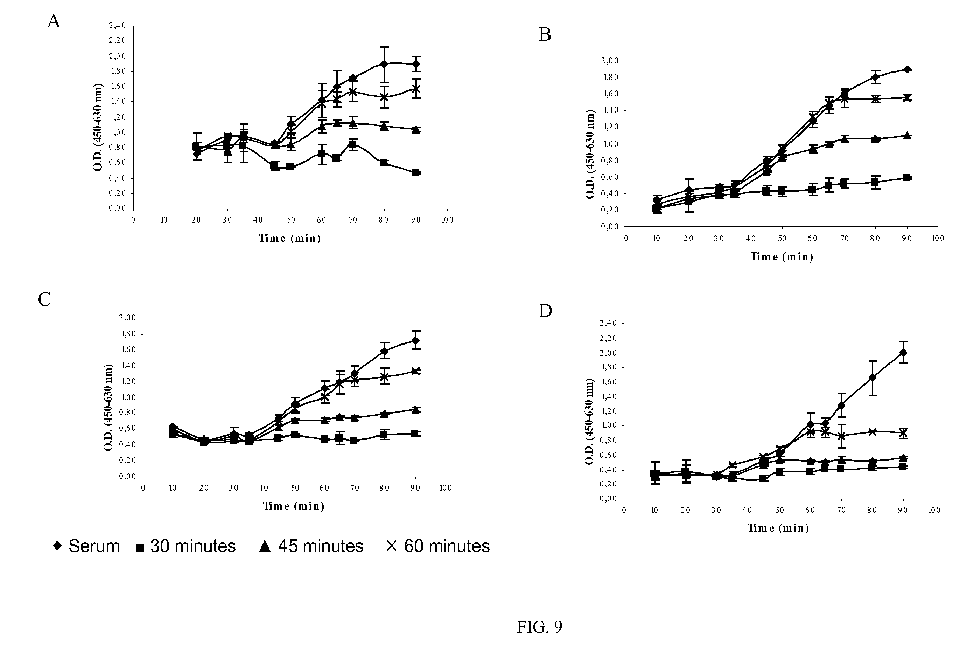

FIG. 9

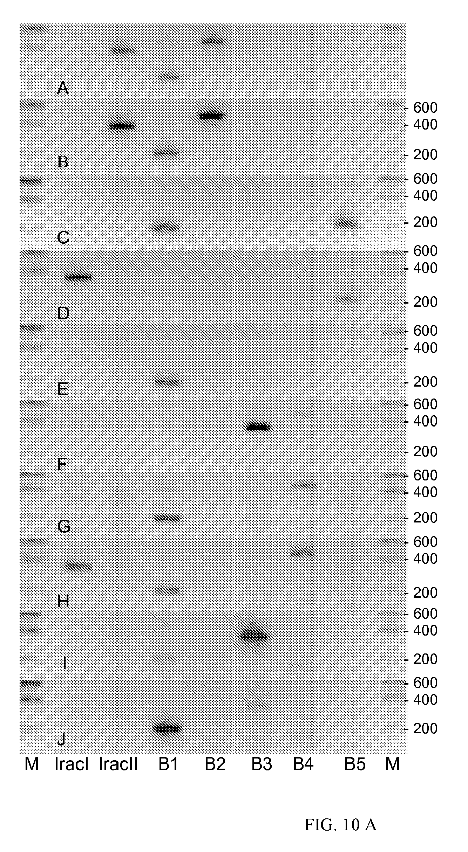

FIG. 10 A

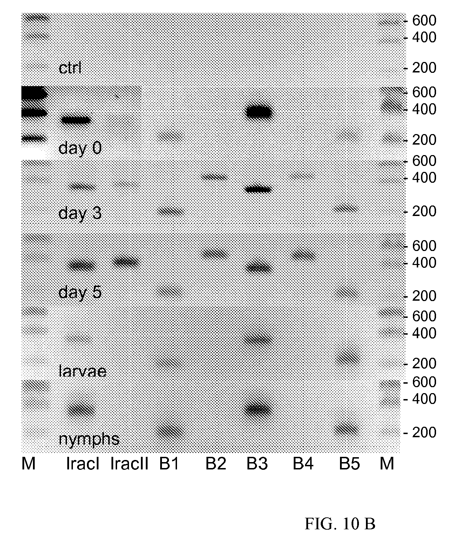

FIG. 10 B

116.3 ——
97.4 ——
66.3 ——
55.4 ——
36.5 ——
31.0 ——
21.5 ——

FIG. 11 A

FIG. 11 B

| European Patent Office | EUROPEAN SEARCH REPORT | Application Number<br>EP 07 11 9924 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/002734 A (HENOGEN S A [BE]; DAIX VIRGINIE [BE]; GODFROID EDMOND [BE]; PASTORET P) 9 January 2003 (2003-01-09) *Whole document, in particular Abstract and claims* | 1-19 | INV.<br>C07K14/435<br>C12N15/12<br>C12N15/63<br>C07K16/18<br>A61K38/17<br>A61K39/395 |
| X | EP 1 270 723 A (HENOGEN S A [BE]) 2 January 2003 (2003-01-02) *Whole document, in particular Abstract and claims* | 1-19 | |
| X | WO 00/77198 A (HENOGEN S A [BE]; GODFROID EDMOND [BE]; BOLLEN ALEX [BE]; LEBOULLE GER) 21 December 2000 (2000-12-21) *Whole document, in particular Abstract and claims* | 1-19 | |
| X,D | DAIX V ET AL: "Ixodes ticks belonging to the Ixodes ricinus complex encode a family of anticomplement proteins" INSECT MOLECULAR BIOLOGY, vol. 16, no. 2, April 2007 (2007-04), pages 155-166, XP002481978 ISSN: 0962-1075 *Whole document, in particular Abstract, Discussion, table 1 and figures 1 and 6* | 1-19 | TECHNICAL FIELDS SEARCHED (IPC)<br>C07K<br>C12N<br>A61K |
| A | LAWRIE C H ET AL: "IXODES TICKS: SERUM SPECIES SENSITIVITY OF ANTICOMPLEMENT ACTIVITY" EXPERIMENTAL PARASITOLOGY, NEW YORK, NY, US, vol. 93, no. 4, 1 December 1999 (1999-12-01), pages 207-214, XP001011316 ISSN: 0014-4894 *Whole document* | | |

-/--

The present search report has been drawn up for all claims

| Place of search | Date of completion of the search | Examiner |
|---|---|---|
| Munich | 28 May 2008 | Bulcao de Melo B., T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

**European Patent Office**

**EUROPEAN SEARCH REPORT**

Application Number

EP 07 11 9924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | VALENZUELA J G ET AL: "Purification, cloning, and expression of a novel salivary anticomplement protein from the tick, Ixodes scapularis" JOURNAL OF BIOLOGICAL CHEMISTRY, AL, vol. 275, no. 25, 23 June 2000 (2000-06-23), pages 18717-18723, XP002182306 ISSN: 0021-9258 *Whole document, in particular Abstract* ----- | | |

TECHNICAL FIELDS SEARCHED (IPC)

The present search report has been drawn up for all claims

| Place of search | Date of completion of the search | Examiner |
|---|---|---|
| Munich | 28 May 2008 | Bulcao de Melo B., T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

EP 2 055 715 A1

**European Patent Office**

**LACK OF UNITY OF INVENTION SHEET B**

Application Number

EP 07 11 9924

The Search Division considers that the present European patent application does not comply with the requirements of unity of invention and relates to several inventions or groups of inventions, namely:

1. claims: 1-6 (completely) and 7-19 (partially)

> Invention 1 concerns an anticomplement polypeptide having SEQ ID NO:1. Invention 1 further concerns a vector, a cell, pharmaceutical and immunological compositions and a diagnostic kit, all relating to said polypeptide.
> ---

2. claims: 7-19 (partially)

> Invention 2 concerns an anticomplement polypeptide having SEQ ID NO:2. Invention 2 further concerns a vector, a cell, pharmaceutical and immunological compositions and a diagnostic kit, all relating to said polypeptide.
> ---

3. claims: 7-19 (partially)

> Invention 3 concerns an anticomplement polypeptide having SEQ ID NO:3. Invention 3 further concerns a vector, a cell, pharmaceutical and immunological compositions and a diagnostic kit, all relating to said polypeptide.
> ---

4. claims: 7-19 (partially)

> Invention 4 concerns an anticomplement polypeptide having SEQ ID NO:4. Invention 4 further concerns a vector, a cell, pharmaceutical and immunological compositions and a diagnostic kit, all relating to said polypeptide.
> ---

5. claims: 7-19 (partially)

> Invention 5 concerns an anticomplement polypeptide having SEQ ID NO:5. Invention 5 further concerns a vector, a cell, pharmaceutical and immunological compositions and a diagnostic kit, all relating to said polypeptide.
> ---

**ANNEX TO THE EUROPEAN SEARCH REPORT
ON EUROPEAN PATENT APPLICATION NO.**                    EP 07 11 9924

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2008

| Patent document cited in search report | | Publication date | Patent family member(s) | | Publication date |
|---|---|---|---|---|---|
| WO 03002734 | A | 09-01-2003 | NONE | | |
| EP 1270723 | A | 02-01-2003 | NONE | | |
| WO 0077198 | A | 21-12-2000 | AT | 374818 T | 15-10-2007 |
| | | | AU | 5055800 A | 02-01-2001 |
| | | | CA | 2376421 A1 | 21-12-2000 |
| | | | EP | 1187916 A2 | 20-03-2002 |
| | | | ES | 2293900 T3 | 01-04-2008 |
| | | | JP | 2003502039 T | 21-01-2003 |
| | | | US | 2002127235 A1 | 12-09-2002 |

EPO FORM P0459

For more details about this annex : see Official Journal of the European Patent Office, No. 12/82

## REFERENCES CITED IN THE DESCRIPTION

### Patent documents cited in the description

- EP 671948 A
- WO 2007068907 A
- WO 2006123155 A
- US 6544518 B
- WO 0230458 A

### Non-patent literature cited in the description

- JANEWAY CA JR et al. MJ Immunobiology. Garland Publishing, 2001
- HOURCADE D et al. *Adv Immunol,* 1989, vol. 45, 381-416
- FEARON DT ; AUSTEN KF. *J Exp Med.,* 1975, vol. 142, 856-63
- PANGBURN MK. *J immunol,* 1989, vol. 142, 202-207
- SMITH CA et al. *J Biol Chem,* 1984, vol. 259, 4582-4588
- GOUNDIS D ; REID KBM. *Nature,* 1988, vol. 335, 82-85
- NOLAN KF et al. *Eur J Immunol,* 1991, vol. 21, 771-776
- SMITH KF et al. *Biochemistry,* 1991, vol. 30, 8000-8008
- DISCIPIO RG. *Biochem J.,* 1981, vol. 199, 485-496
- HOURCADE DE. *J Biol Chem,* 2006, vol. 281, 2128-2132
- GUPTA-BANSAL R et al. *Mol Immunol.,* 2000, vol. 37, 191-201
- JOINER KA. *Ann Rev Microbiol,* 1998, vol. 42, 201-230
- WIKEL SK ; ALLEN JR. *Immunology,* 1977, vol. 32, 457-465
- WIKEL SK. *Am J Trop Med Hyg.,* 1979, vol. 28, 586-590
- RIBEIRO JM. *Exp Parasitol.,* 1987, vol. 64, 347-353
- LAWRIE CH et al. *Exp Parasitol,* 1999, vol. 93, 207-214
- VALENZUELA JG et al. *J Biol Chem,* 2000, vol. 275, 18717-18723
- MEJRI N et al. *Parasitol Res,* 2002, vol. 88, 192-197
- SOARES CA et al. *Insect Mol Biol,* 2005, vol. 14, 443-452
- DAS S, FS et al. *J Infect Dis,* 2001, vol. 184, 1056-1064
- TYSON K et al. *Insect Molecular Biology,* 2007, vol. 16, 469-479
- FRANCISCHETTI IM, JF et al. *Insect Biochem Mol Biol.,* 2005, vol. 35, 1142-1161
- DAIX V et al. *Insect Mol Biol.,* 2007, vol. 16, 155-166
- ASTIGARRAGA A et al. *Parasite Immunol.,* 1997, vol. 19, 401-410
- NUNN MA et al. *J Immunol,* 2005, vol. 174, 2084-2091
- VALENZUELA JG et al. *J Exp Biol,* 2002, vol. 205 (18), 2843-2864
- RIBEIRO JM et al. *Insect Biochem Mol Biol,* 2006, vol. 36, 111-129
- ULLMANN AJ et al. *Insect Mol Biol,* 2005, vol. 4, 217-222
- PALMER MJ et al. *Insect Mol Biol,* 1994, vol. 3, 57-62
- LAWRIE CH et al. *Mol Immunol.,* 2005, vol. 42, 31-38
- CURMAN B et al. *Biochemistry,* 1977, vol. 16, 5368-5375
- KORDIS D et al. *Gene,* 2000, vol. 261, 43-52
- ZUPUNSKI V et al. *FEBS Lett,* 2003, vol. 547, 131-136
- MANS BJ et al. *Mol Biol Evol,* 2002, vol. 19, 1695-1705
- LANZARO GC et al. *Insect Mol Biol.,* 1999, vol. 8, 267-275
- MILLERON RS et al. *Am J Trop Med Hyg,* 2004, vol. 70, 286-293
- MILLERON RS et al. *Am J Trop Med Hyg,* 2004, vol. 70, 278-285
- SCHREIBER RD et al. *J Exp Med,* 1978, vol. 148, 1722-1727
- PANGBURN MK et al. *Biochem J,* 1986, vol. 235, 723-730
- FINLAY BB et al. *Cell,* 2006, vol. 124, 767-782
- KOSTAVASILI I et al. *J Immunol.,* 1997, vol. 158, 1763-1771
- CHUNG KM et al. *Proc Natl Acad Sci U S A.,* 2006, vol. 103, 19111-191116

- **REID KB ; DAY AJ.** *Immunol. Today,* 1989, vol. 10, 177-180
- **ANDERSON JF.** *Scand J Infect Dis,* 1991, vol. 77, 3-34
- **MILNE A.** *Parasitology,* 1949, vol. 39, 173-197
- **HOOGSTRAAL H ; AESCHLIMANN A.** *Bulletin de la société entomologique suisse,* 1982, vol. 55, 5-32
- **SCHROEDER H et al.** *Microbes Infect,* 2007, vol. 9, 247-250
- **NONAKA M ; KIMURA A.** *Immunogenetics,* 2006, vol. 58, 701-713
- **HOLERS V.M. ; THURMAN JM.** *Mol Immunol,* 2004, vol. 41 (2-3), 147-52
- **THE INVENTORSISMAN HF et al.** *Trans Assoc Am Physicians,* 1990, vol. 103, 64-72
- **THOMAS TC et al.** *Mol Immunol,* 1996, vol. 33, 1389-401
- **KOZAK M.** *J Cell Biol,* 1989, vol. 108, 229-241
- **PAESEN GC et al.** *Mol Cell,* 1999, vol. 3, 661-671
- **PREVOT PP et al.** *J Biol Chem.,* 2006, vol. 281, 26361-26369
- **EMANUELSSON O et al.** *Protocols,* 2007, vol. 2, 953-971
- **JULENIUS K et al.** *Glycobiology,* 2005, vol. 15, 153-164
- **KROGH A et al.** *J Mol Biol,* 2001, vol. 305, 567-580
- **VULLO A et al.** *Bioinformatics,* 2004, vol. 20, 653-659
- **GABORIAUD C et al.** *FEBS Lett,* 1987, vol. 224, 149-155
- **CALLEBAUT I et al.** *Cell Mol Life Sci.,* 1997, vol. 53, 621-645
- **NICHOLAS KB et al.** *EMBNEW NEWS,* 1997, vol. 4, 14
- **GUINDON S et al.** *Systematic Biology,* 2003, vol. 52, 696-704
- **PAGE RD.** *Comput Appl Biosci.,* 1996, vol. 12, 357-358
- **CAI W ; PEI J ; GRISHIN NV.** *BMC Evol Biol.,* 2004, vol. 4, 33
- **KUMAR S et al.** *Brief Bioinform.,* 2004, vol. 5, 150-163
- **GICLAS PC et al.** Manual of clinical laboratory immunology. ASM Press, 1997, 181-186